# EUROPEAN PATENT APPLICATION

(11) **EP 4 290 596 A1**
(43) Date of publication of application: **13.12.2023**
(21) Application number: 22749727.8
(22) Date of filing: 02.02.2022
(51) Int. Cl.: H01L 51/42, C07D 519/00, H01L 27/146, H01L 27/30

(54) **PHOTOELECTRIC CONVERSION ELEMENT, IMAGING ELEMENT, PHOTOSENSOR, AND COMPOUND**

(30) Priority: 05.02.2021 JP 2021017634; 09.07.2021 JP 2021114243
(71) Applicant: FUJIFILM Corporation, Tokyo 106-8620 (JP)
(72) Inventor: KANEKO, Kazuhei, Ashigarakami-gun, Kanagawa 258-8577 (JP); YAMAMOTO, Yosuke, Ashigarakami-gun, Kanagawa 258-8577 (JP)
(74) Representative: HGF
(86) International application number: PCT/JP2022/004012
(87) International publication number: WO 2022/168856

(57) **Abstract**

The present invention is to provide a photoelectric conversion element having an excellent photoelectric conversion efficiency for light in a wide wavelength range and excellent manufacturing suitability. In addition, an imaging element, an optical sensor, and a compound are provided. A light source conversion element includes a conductive film, a photoelectric conversion film, and a transparent conductive film in this order, and the photoelectric conversion film contains a compound represented by Formula (1).

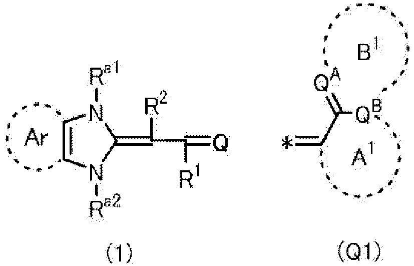

## Description

### BACKGROUND OF THE INVENTION

### 1. Field of the Invention

The present invention relates to a photoelectric conversion element, an imaging element, an optical sensor, and a compound.

### 2. Description of the Related Art

In recent years, development of an element having a photoelectric conversion film has progressed.

For example, it is disclosed in WO2020/013246A that a photoelectric conversion element includes a conductive film, a photoelectric conversion film, and a transparent conductive film in this order, and the photoelectric conversion film contains a compound represented by Formula (1).

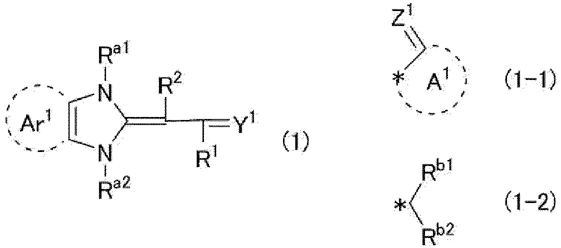

### SUMMARY OF THE INVENTION

Various characteristics are required for a photoelectric conversion element. For example, in recent years, a photoelectric conversion film having favorable photoelectric conversion characteristics for light in a wide wavelength range has also been required.

The present inventor has produced a photoelectric conversion element by using the compound disclosed in WO2020/013246A, and evaluated the obtained photoelectric conversion element. As a result, it was found that there was room for improvement in that the photoelectric conversion efficiency with respect to light in a wide wavelength range (for example, 500 to 600 nm).

In addition, the photoelectric conversion element is required to have excellent manufacturing appropriateness such that the photoelectric conversion characteristics do not deteriorate even in a case where the vapor deposition rate during the formation of the photoelectric conversion film is increased, in order to meet the requirements for manufacturing a product.

In view of the above circumstances, an object of the present invention is to provide a photoelectric conversion element having an excellent photoelectric conversion efficiency for light in a wide wavelength range and excellent manufacturing suitability.

Another object of the present invention is to provide an imaging element and an optical sensor which include the photoelectric conversion element. Furthermore, an object of the present invention is also to provide a compound applied to the photoelectric conversion element.

The inventors of the present invention have conducted extensive studies on the above-described problems. As a result, the inventors have found that it is possible to solve the above-described problems by applying the compound having a predetermined structure to the photoelectric conversion film, and have completed the present invention.

Specifically, the present invention has been completed by adopting the following configuration.

[1] A photoelectric conversion element comprising, in the following order:
   a conductive film;
   a photoelectric conversion film; and
   a transparent conductive film,
   in which the photoelectric conversion film contains a compound represented by Formula (1), 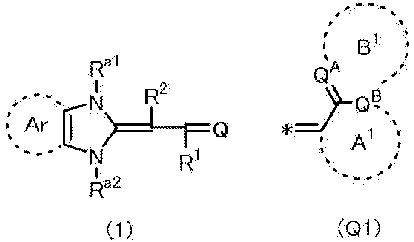
   in Formula (1), Ar represents an aromatic ring which may have a substituent,
   R¹ and R² each independently represent a hydrogen atom or a substituent,
   R^{a1} and R^{a2} each independently represent an aryl group which may have a substituent, -C(R^{L1})(R^{L2})(R^{L3}), or a heteroaryl group which may have a substituent,
   R^{L1} to R^{L3} each independently represent an alkyl group which may have a substituent, an aryl group which may have a substituent, a heteroaryl group which may have a substituent, or a hydrogen atom, and at least two of R^{L1}, R^{L2}, or R^{L3} each independently represent an alkyl group which may have a substituent, an aryl group which may have a substituent, or a heteroaryl group which may have a substituent, an alkyl group which may have a substituent, an aryl group which may have a substituent, and a heteroaryl group which may have a substituent, which are represented by R^{L1} to R^{L3}, may be bonded to each other to form a ring, and
   Q represents a group represented by Formula (Q1),
   in Formula (Q1), * represents a bonding position,
   Q^{A} represents a nitrogen atom or -CQ^{X}=, Q^{x} represents a hydrogen atom or a substituent,
   Q^{B} represents a nitrogen atom, a group represented by Formula (C), or -CQ^{Y}<, Q^{y} represents a hydrogen atom or a substituent,
   A¹ represents a ring which at least contains two carbon atoms and Q^{B}, and may have a substituent, and
   B¹ represents a ring which at least contains one carbon atom, Q^{A}, and Q^{B}, and may have a substituent, 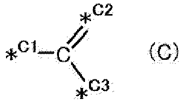
   in Formula (C), *^{C1} to *^{C3} each represent a bonding position.
[2] The photoelectric conversion element according to [1], in which the compound represented by Formula (1) is a compound represented by Formula (2), 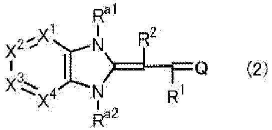
   in Formula (2), R¹ and R² each independently represent a hydrogen atom or a substituent,
   R^{a1} and R^{a2} each independently represent an aryl group which may have a substituent, -C(R^{L1})(R^{L2})(R^{L3}), or a heteroaryl group which may have a substituent,
   R^{L1} to R^{L3} each independently represent an alkyl group which may have a substituent, an aryl group which may have a substituent, a heteroaryl group which may have a substituent, or a hydrogen atom, and at least two of R^{L1}, R^{L2}, or R^{L3} each independently represent an alkyl group which may have a substituent, an aryl group which may have a substituent, or a heteroaryl group which may have a substituent, an alkyl group which may have a substituent, an aryl group which may have a substituent, and a heteroaryl group which may have a substituent, which are represented by R^{L1} to R^{L3}, may be bonded to each other to form a ring,
   Q represents the group represented by Formula (Q1),
   X¹ to X⁴ each independently represent a nitrogen atom or -CR^{c1}=,
   R^{c1} represents a hydrogen atom or a substituent, and
   in a case where a plurality of R^{c1}'s exist, the plurality of R^{c1}'s may be bonded to each other to form a ring.
[3] The photoelectric conversion element according to [1] or [2], in which the compound represented by Formula (1) is a compound represented by Formula (3), 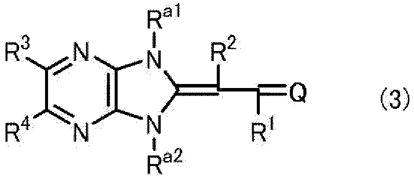
   in Formula (3), R¹ to R⁴ each independently represent a hydrogen atom or a substituent,
   R³ and R⁴ may be bonded to each other to form a ring,
   R^{a1} and R^{a2} each independently represent an aryl group which may have a substituent, -C(R^{L1})(R^{L2})(R^{L3}), or a heteroaryl group which may have a substituent,
   R^{L1} to R^{L3} each independently represent an alkyl group which may have a substituent, an aryl group which may have a substituent, a heteroaryl group which may have a substituent, or a hydrogen atom, and at least two of R^{L1}, R^{L2}, or R^{L3} each independently represent an alkyl group which may have a substituent, an aryl group which may have a substituent, or a heteroaryl group which may have a substituent, an alkyl group which may have a substituent, an aryl group which may have a substituent, and a heteroaryl group which may have a substituent, which are represented by R^{L1} to R^{L3}, may be bonded to each other to form a ring, and
   Q represents the group represented by Formula (Q1).
[4] The photoelectric conversion element according to any one of [1] to [3], in which the compound represented by Formula (1) is a compound represented by Formula (4), 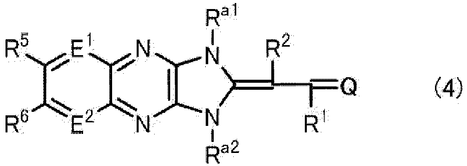
   in Formula (4), R¹, R², R⁵, and R⁶ each independently represent a hydrogen atom or a substituent,
   R⁵ and R⁶ may be bonded to each other to form a ring,
   R^{a1} and R^{a2} each independently represent an aryl group which may have a substituent, -C(R^{L1})(R^{L2})(R^{L3}), or a heteroaryl group which may have a substituent,
   R^{L1} to R^{L3} each independently represent an alkyl group which may have a substituent, an aryl group which may have a substituent, a heteroaryl group which may have a substituent, or a hydrogen atom, and at least two of R^{L1}, R^{L2}, or R^{L3} each independently represent an alkyl group which may have a substituent, an aryl group which may have a substituent, or a heteroaryl group which may have a substituent, an alkyl group which may have a substituent, an aryl group which may have a substituent, and a heteroaryl group which may have a substituent, which are represented by R^{L1} to R^{L3}, may be bonded to each other to form a ring,
   Q represents the group represented by Formula (Q1),
   E¹ represents a nitrogen atom or -CR^{E1}=, R^{E1} represents a hydrogen atom or a substituent,
   E² represents a nitrogen atom or -CR^{E2}=, and R^{E2} represents a hydrogen atom or a substituent.
[5] The photoelectric conversion element according to any one of [1] to [4], in which Q represents a group represented by Formula (Q2), 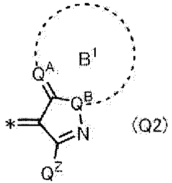
   in Formula (Q2), * represents a bonding position,
   Q^{A} represents a nitrogen atom or -CQ^{X}=, Q^{x} represents a hydrogen atom or a substituent,
   Q^{B} represents a nitrogen atom, the group represented by Formula (C), or -CQ^{Y}<, Q^{y} represents a hydrogen atom or a substituent,
   Q^{Z} represents a hydrogen atom or a substituent, and
   B¹ represents a ring which at least contains one carbon atom, Q^{A}, and Q^{B}, and may have a substituent.
[6] The photoelectric conversion element according to any one of [1] to [5], in which Q represents a group represented by Formula (Q3), 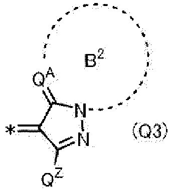
   in Formula (Q3), * represents a bonding position,
   Q^{A} represents a nitrogen atom or -CQ^{X}=, Q^{x} represents a hydrogen atom or a substituent,
   Q^{Z} represents a hydrogen atom or a substituent, and
   B² represents a ring which at least contains one carbon atom, one nitrogen atom, and Q^{A}, and may have a substituent.
[7] The photoelectric conversion element according to any one of [1] to [6], in which the photoelectric conversion film further includes a n-type organic semiconductor, and
   the photoelectric conversion film has a bulk hetero structure formed in a state where the compound represented by Formula (1) and the n-type organic semiconductor are mixed to each other.
[8] The photoelectric conversion element according to [7], in which the n-type organic semiconductor includes fullerenes selected from the group consisting of a fullerene and a derivative thereof.
[9] The photoelectric conversion element according to [7] or [8], in which the photoelectric conversion film further contains a p-type organic semiconductor.
[10] The photoelectric conversion element according to any one of [1] to [9], further comprising one or more interlayers between the conductive film and the transparent conductive film, in addition to the photoelectric conversion film.
[11] An imaging element comprising the photoelectric conversion element according to any one of [1] to [10].
[12] An optical sensor comprising the photoelectric conversion element according to any one of [1] to [10].
[13] A compound represented by Formula (1), 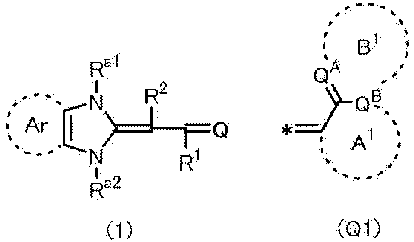
   in Formula (1), Ar represents an aromatic ring which may have a substituent,
   R¹ and R² each independently represent a hydrogen atom or a substituent,
   R^{a1} and R^{a2} each independently represent an aryl group which may have a substituent, -C(R^{L1})(R^{L2})(R^{L3}), or a heteroaryl group which may have a substituent,
   R^{L1} to R^{L3} each independently represent an alkyl group which may have a substituent, an aryl group which may have a substituent, a heteroaryl group which may have a substituent, or a hydrogen atom, and at least two of R^{L1}, R^{L2}, or R^{L3} each independently represent an alkyl group which may have a substituent, an aryl group which may have a substituent, or a heteroaryl group which may have a substituent, an alkyl group which may have a substituent, an aryl group which may have a substituent, and a heteroaryl group which may have a substituent, which are represented by R^{L1} to R^{L3}, may be bonded to each other to form a ring, and
   Q represents a group represented by Formula (Q1),
   in Formula (Q1), * represents a bonding position,
   Q^{A} represents a nitrogen atom or -CQ^{X}=, Q^{x} represents a hydrogen atom or a substituent,
   Q^{B} represents a nitrogen atom, a group represented by Formula (C), or -CQ^{Y}<, Q^{y} represents a hydrogen atom or a substituent,
   A¹ represents a ring which at least contains two carbon atoms and Q^{B}, and may have a substituent, and
   B¹ represents a ring which at least contains one carbon atom, Q^{A}, and Q^{B}, and may have a substituent, 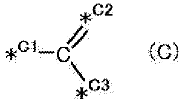
   in Formula (C), *^{C1} to *^{C3} each represent a bonding position.
[14] The compound according to [13], in which the compound represented by Formula (1) is a compound represented by Formula (2), 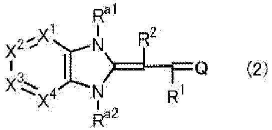
   in Formula (2), R¹ and R² each independently represent a hydrogen atom or a substituent,
   R^{a1} and R^{a2} each independently represent an aryl group which may have a substituent, -C(R^{L1})(R^{L2})(R^{L3}), or a heteroaryl group which may have a substituent,
   R^{L1} to R^{L3} each independently represent an alkyl group which may have a substituent, an aryl group which may have a substituent, a heteroaryl group which may have a substituent, or a hydrogen atom, and at least two of R^{L1}, R^{L2}, or R^{L3} each independently represent an alkyl group which may have a substituent, an aryl group which may have a substituent, or a heteroaryl group which may have a substituent, an alkyl group which may have a substituent, an aryl group which may have a substituent, and a heteroaryl group which may have a substituent, which are represented by R^{L1} to R^{L3}, may be bonded to each other to form a ring,
   Q represents the group represented by Formula (Q1),
   X¹ to X⁴ each independently represent a nitrogen atom or -CR^{c1}=,
   R^{c1} represents a hydrogen atom or a substituent, and
   in a case where a plurality of R^{c1}'s exist, the plurality of R^{c1}'s may be bonded to each other to form a ring.
[15] The compound according to [13] or [14], in which the compound represented by Formula (1) is a compound represented by Formula (3), 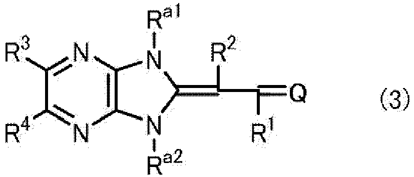
   in Formula (3), R¹ to R⁴ each independently represent a hydrogen atom or a substituent,
   R³ and R⁴ may be bonded to each other to form a ring,
   R^{a1} and R^{a2} each independently represent an aryl group which may have a substituent, -C(R^{L1})(R^{L2})(R^{L3}), or a heteroaryl group which may have a substituent,
   R^{L1} to R^{L3} each independently represent an alkyl group which may have a substituent, an aryl group which may have a substituent, a heteroaryl group which may have a substituent, or a hydrogen atom, and at least two of R^{L1}, R^{L2}, or R^{L3} each independently represent an alkyl group which may have a substituent, an aryl group which may have a substituent, or a heteroaryl group which may have a substituent, an alkyl group which may have a substituent, an aryl group which may have a substituent, and a heteroaryl group which may have a substituent, which are represented by R^{L1} to R^{L3}, may be bonded to each other to form a ring, and
   Q represents the group represented by Formula (Q1).
[16] The compound according to any one of [13] to [15], in which the compound represented by Formula (1) is a compound represented by Formula (4), 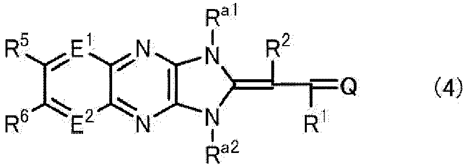
   in Formula (4), R¹, R², R⁵, and R⁶ each independently represent a hydrogen atom or a substituent,
   R⁵ and R⁶ may be bonded to each other to form a ring,
   R^{a1} and R^{a2} each independently represent an aryl group which may have a substituent, -C(R^{L1})(R^{L2})(R^{L3}), or a heteroaryl group which may have a substituent,
   R^{L1} to R^{L3} each independently represent an alkyl group which may have a substituent, an aryl group which may have a substituent, a heteroaryl group which may have a substituent, or a hydrogen atom, and at least two of R^{L1}, R^{L2}, or R^{L3} each independently represent an alkyl group which may have a substituent, an aryl group which may have a substituent, or a heteroaryl group which may have a substituent, an alkyl group which may have a substituent, an aryl group which may have a substituent, and a heteroaryl group which may have a substituent, which are represented by R^{L1} to R^{L3}, may be bonded to each other to form a ring,
   Q represents the group represented by Formula (Q1),
   E¹ represents a nitrogen atom or -CR^{E1}=, R^{E1} represents a hydrogen atom or a substituent,
   E² represents a nitrogen atom or -CR^{E2}=, and R^{E2} represents a hydrogen atom or a substituent.
[17] The compound according to any one of [13] to [16], in which Q represents a group represented by Formula (Q2), 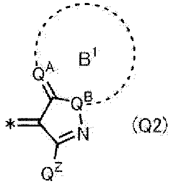
   in Formula (Q2), * represents a bonding position,
   Q^{A} represents a nitrogen atom or -CQ^{X}=, Q^{x} represents a hydrogen atom or a substituent,
   Q^{B} represents a nitrogen atom, the group represented by Formula (C), or -CQ^{Y}<, Q^{y} represents a hydrogen atom or a substituent,
   Q^{Z} represents a hydrogen atom or a substituent, and
   B¹ represents a ring which at least contains one carbon atom, Q^{A}, and Q^{B}, and may have a substituent.
[18] The compound according to any one of [13] to [17], in which Q represents a group represented by Formula (Q3), 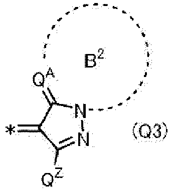
   in Formula (Q3), * represents a bonding position,
   Q^{A} represents a nitrogen atom or -CQ^{X}=, Q^{x} represents a hydrogen atom or a substituent,
   Q^{Z} represents a hydrogen atom or a substituent, and
   B² represents a ring which at least contains one carbon atom, one nitrogen atom, and Q^{A}, and may have a substituent.

According to the present invention, it is possible to provide the photoelectric conversion element having an excellent photoelectric conversion efficiency for light in a wide wavelength range and excellent manufacturing suitability.

According to the present invention, it is possible to provide the imaging element and the optical sensor which include the photoelectric conversion element. Furthermore, according to the present invention, it is possible to provide the compound applied to the photoelectric conversion element.

### BRIEF DESCRIPTION OF THE DRAWINGS

Fig. 1 is a schematic cross-sectional view illustrating a configuration example of a photoelectric conversion element.
Fig. 2 is a schematic cross-sectional view illustrating a configuration example of the photoelectric conversion element.

### DESCRIPTION OF THE PREFERRED EMBODIMENTS

Hereinafter, suitable embodiments of a photoelectric conversion element of the present invention will be described.

In the present specification, a "substituent" includes a group exemplified by a substituent W described later, unless otherwise specified.

### (Substituent W)

A substituent W in the present specification will be described below.

Examples of the substituent W include a halogen atom (a fluorine atom, a chlorine atom, a bromine atom, an iodine atom, and the like), an alkyl group (including a cycloalkyl group, a bicycloalkyl group, and a tricycloalkyl group), an alkenyl group (including a cycloalkenyl group and a bicycloalkenyl group), an alkynyl group, an aryl group, a heteroaryl group (may also be referred to as a heterocyclic group), a cyano group, a nitro group, an alkoxy group, an aryloxy group, a silyloxy group, a heterocyclic oxy group, an acyloxy group, a carbamoyloxy group, an alkoxycarbonyloxy group, an aryloxycarbonyloxy group, a secondary or tertiary amino group (including an anilino group), an alkylthio group, an arylthio group, a heterocyclic thio group, an alkyl or an arylsulfinyl group, an alkyl or an arylsulfonyl group, an acyl group, an aryloxycarbonyl group, an alkoxycarbonyl group, an aryl or a heterocyclic azo group, an imide group, a phosphino group, a phosphinyl group, a phosphinyloxy group, a phosphinylamino group, a phosphono group, a silyl group, a carboxy group, a phosphoric acid group, a sulfonic acid group, a hydroxy group, a thiol group, an acylamino group, a carbamoyl group, a ureido group, a boronic acid group, and a primary amino group. Each of the above-described groups may further have a substituent (for example, one or more groups of each of the above-described groups), as possible. For example, an alkyl group which may have a substituent is also included as a form of the substituent W.

In addition, in a case where the substituent W has a carbon atom, the number of carbon atoms of the substituent W is, for example, 1 to 20.

The number of atoms other than a hydrogen atom included in the substituent W is, for example, 1 to 30.

In addition, the specific compound preferably does not contain, as a substituent, a carboxy group, a salt of a carboxy group, a salt of a phosphoric acid group, a sulfonic acid group, a salt of a sulfonic acid group, a hydroxy group, a thiol group, an acylamino group, a carbamoyl group, a ureido group, or a boronic acid group (-B(OH)₂) and/or a primary amino group.

In the present specification, examples of the halogen atom include a fluorine atom, a chlorine atom, a bromine atom, and an iodine atom.

In the present specification, unless otherwise specified, the number of carbon atoms of the alkyl group is preferably 1 to 20, more preferably 1 to 10, and still more preferably 1 to 6.

The alkyl group may be linear, branched, or cyclic.

Examples of the alkyl group include a methyl group, an ethyl group, a n-propyl group, an i-propyl group, a n-butyl group, a t-butyl group, a n-hexyl group, a cyclopentyl group, and other alkyl groups.

In addition, the alkyl group may be, for example, a cycloalkyl group, a bicycloalkyl group, or a tricycloalkyl group, and may have a cyclic structure thereof as a partial structure.

In the alkyl group which may have a substituent, a substituent which may be contained in the alkyl group is not particularly limited, an example thereof includes the substituent W, and an aryl group (preferably having 6 to 18 carbon atoms, and more preferably having 6 carbon atoms), a heteroaryl group (preferably having 5 to 18 carbon atoms, and more preferably having 5 and 6 carbon atoms), or a halogen atom (preferably a fluorine atom or a chlorine atom) is preferable.

In the present specification, unless otherwise specified, the above-described alkyl group is preferable as an alkyl group moiety in the alkoxy group. The alkyl group moiety in the alkylthio group is preferably the above-described alkyl group.

In the alkoxy group which may have a substituent, the substituent which may be contained in the alkoxy group includes the same examples as the substituent in the alkyl group which may have a substituent. In the alkylthio group which may have a substituent, the substituent which may be contained in the alkylthio group includes the same examples as the substituent in the alkyl group which may have a substituent.

In the present specification, the alkenyl group may be any of linear, branched, or cyclic, unless otherwise specified. The alkenyl group preferably has 2 to 20 carbon atoms. In the alkenyl group which may have a substituent, the substituent which may be contained in the alkenyl group includes the same examples as the substituent in the alkyl group which may have a substituent.

In the present specification, an alkynyl group may be any of linear, branched, or cyclic, unless otherwise specified. The alkynyl group preferably has 2 to 20 carbon atoms. In the alkynyl group which may have a substituent, the substituent which may be contained in the alkynyl group includes the same examples as the substituent in the alkyl group which may have a substituent.

In the present specification, unless otherwise specified, examples of a silyl group which may have a substituent include a group represented by -Si(R^{S1})(R^{S2})(R^{S3}). R^{S1}, R^{S2}, and R^{S3} each independently represent a hydrogen atom or a substituent, and preferably represent an alkyl group which may have a substituent, an alkoxy group which may have a substituent, an alkylthio group which may have a substituent, an aryl group which may have a substituent, or a heteroaryl group which may have a substituent.

In the present specification, unless otherwise specified, an aromatic ring may be monocyclic or polycyclic (for example, with 2 to 6 rings). The monocyclic aromatic ring is an aromatic ring having only one aromatic ring structure as a ring structure. The polycyclic (for example, 2 to 6 rings) aromatic ring is an aromatic ring formed of a plurality of (for example, 2 to 6) aromatic ring structures fused as a ring structure.

The number of ring member atoms of the aromatic ring is preferably 5 to 15.

The aromatic ring may be an aromatic hydrocarbon ring or an aromatic heterocyclic ring.

In a case where the aromatic ring is an aromatic heterocyclic ring, the number of heteroatoms contained as ring member atoms is, for example, 1 to 10. Examples of the heteroatoms include a nitrogen atom, a sulfur atom, an oxygen atom, a selenium atom, a tellurium atom, a phosphorus atom, a silicon atom, and a boron atom.

Examples of the aromatic hydrocarbon ring include a benzene ring, a naphthalene ring, an anthracene ring, and a phenanthrene ring.

Examples of the aromatic heterocyclic ring include a pyridine ring, a pyrimidine ring, a pyridazine ring, a pyrazine ring, a triazine ring (1,2,3-triazine ring, 1,2,4-triazine ring, 1,3,5-triazine ring, or the like), and a tetrazine ring (1,2,4,5-tetrazine ring or the like), a quinoxaline ring, a pyrrole ring, a furan ring, a thiophene ring, an imidazole ring, an oxazole ring, a thiazole ring, a benzopyrrole ring, a benzofuran ring, a benzothiophene ring, a benzoimidazole ring, a benzoxazole ring, a benzothiazole ring, a naphthopyrrole ring, a naphthofuran ring, a naphthothiophene ring, a naphthimidazole ring, a naphthoxazole ring, a 3H-pyrrolidine ring, a pyrroloimidazole ring (a 5H-pyrrolo[1,2-a]imidazole ring and the like), an imidazooxazole ring (an imidazo[2,1-b]oxazole ring and the like), a thienothiazole ring (a thieno[2,3-d]thiazole ring and the like), a benzothiadiazole ring, a benzodithiophene ring (benzo[1,2-b:4,5-b']dithiophene ring or the like), a thienothiophene ring (thieno[3,2-b]thiophene ring or the like), a thiazolothiazole ring (thiazolo[5,4-d]thiazole ring or the like), a naphthodithiophene ring (naphtho[2,3-b:6,7-b']dithiophene ring, a naphtho[2,1-b:6,5-b']dithiophene ring, a naphtho[1,2-b:5,6-b']dithiophene ring, a 1,8-dithiadicyclopenta[b,g]naphthalene ring, or the like), a benzothienobenzothiophene ring, a dithieno[3,2-b:2',3'-d]thiophene ring, and a 3,4,7,8-tetrathiadicyclopenta[a,e]pentalene ring.

In the aromatic ring which may have a substituent, a kind of the substituent that the aromatic ring may have is not particularly limited, and examples thereof include a substituent W. In a case where the aromatic ring has a substituent, the number of substituents may be 1 or more (for example, 1 to 4).

In the present specification, the term "aromatic ring group" includes, for example, a group obtained by removing one or more hydrogen atoms (for example, 1 to 5) from the aromatic ring.

In the present specification, the term "aryl group" includes, for example, a group obtained by removing one hydrogen atom from a ring corresponding to an aromatic hydrocarbon ring among the above aromatic rings.

In the present specification, the term "heteroaryl group" includes, for example, a group obtained by removing one hydrogen atom from a ring corresponding to an aromatic heterocyclic ring among the above aromatic rings.

In the present specification, the term "arylene group" includes, for example, a group obtained by removing two hydrogen atoms from a ring corresponding to an aromatic hydrocarbon ring among the above aromatic rings.

In the present specification, the term "heteroarylene group" includes, for example, a group obtained by removing two hydrogen atoms from a ring corresponding to an aromatic heterocyclic ring among the above aromatic rings.

In an aromatic ring group which may have a substituent, an aryl group which may have a substituent, a heteroaryl group which may have a substituent, an arylene group which may have a substituent, and a heteroarylene group which may have a substituent, a kind of the substituents that these groups may have is not particularly limited, and examples thereof include a substituent W. In a case where these groups each of which may have a substituent have substituents, the number of substituents may be 1 or more (for example, 1 to 4).

In the present specification, in a case where a plurality of identical symbols indicating a kind or the number of groups are present in Formula (General Formula), which indicates a chemical structure, contents of these plurality of identical symbols indicating a kind or the number of groups are independent of each other, and the contents of the identical symbols may be the same or different from each other unless otherwise specified.

In the present specification, in a case where a plurality of identical groups (alkyl groups or the like) are present in one Formula (General Formula), which indicates a chemical structure, specific contents between these plurality of identical groups are independent of each other, and the specific contents between the plurality of identical groups may be the same or different from each other, unless otherwise specified.

A bonding direction of a divalent group (for example, -CO-O-) described in the present specification is not limited unless otherwise specified. For example, in a case where Y is -CO-O- in the compound represented by General Formula "X-Y-Z", the compound may be "X-O-CO-Z" or may be "X-CO-O-Z".

In the present specification, regarding a compound that may have a geometric isomer (cis-trans isomer), a general formula or a structural formula representing the above compound may be described only in the form of either a cis isomer or a trans isomer for convenience. Even in such a case, unless otherwise specified, the form of the compound is not limited to either the cis isomer or the trans isomer, and the compound may be either the cis isomer or the trans isomer.

In addition, in the present specification, the numerical range represented by "to" means a range including numerical values denoted before and after "to" as a lower limit value and an upper limit value.

In the present specification, in a case where there are plural substituents, linking groups, and the like (hereinafter, referred to as substituents and the like) represented by specific symbols, or a case where a plurality of substituents and the like are specified all together, each of the substituents and the like may be the same or may be different from each other. This also applies to a case of specifying the number of substituents and the like.

In the present specification, a hydrogen atom may be a light hydrogen atom (an ordinary hydrogen atom) or a deuterium atom (a double hydrogen atom and the like).

### [Photoelectric Conversion Element]

The photoelectric conversion element according to an embodiment of the present invention includes a conductive film, a photoelectric conversion film, and a transparent conductive film in this order, in which the photoelectric conversion film contains a compound represented by Formula (1) described later (hereinafter, referred to as a "specific compound").

The mechanism capable of solving the above problems by adopting such a configuration of the photoelectric conversion element according to the embodiment of the present invention is not always clear, but the present inventors have presumed as follows.

That is, the crystallization of the specific compound is suppressed even in a case of being heated because the specific compound has a predetermined nitrogen atom to which relatively large substituents (R^{a1} and R^{a2} in Formula (1)) are bonded. Therefore, it is considered that the photoelectric conversion efficiency less deteriorates even in a case where the vapor deposition rate during the production of the photoelectric conversion film is increased, and the manufacturing suitability is improved. In addition, it is considered that the specific compound can absorb light in a wide wavelength range because an acceptor moiety (the group represented by Q in Formula (1)) has a predetermined structure, thereby improving the photoelectric conversion efficiency for the light in a wide wavelength range.

In addition, the photoelectric conversion element according to the embodiment of the present invention also has favorable responsiveness.

Hereinafter, the fact that the photoelectric conversion efficiency of the photoelectric conversion element for light in a wide wavelength range is more excellent, the manufacturing suitability is more excellent, and/or the responsiveness is more excellent is also referred to as "the effect of the present invention is more excellent".

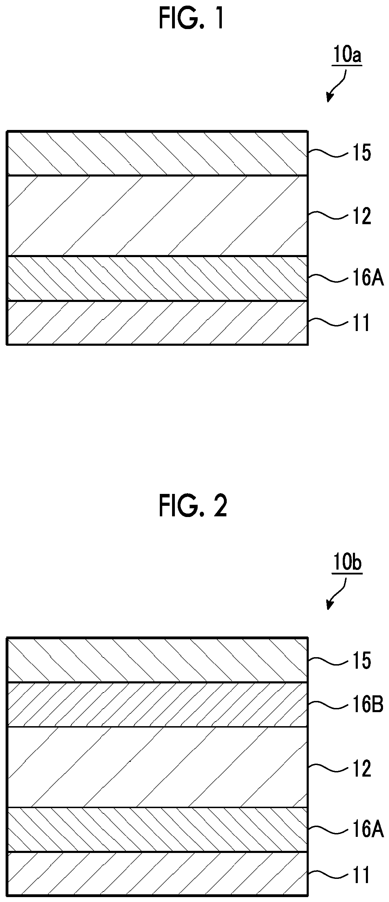

Fig. 1 is a schematic cross-sectional view of one embodiment of a photoelectric conversion element according to the embodiment of the present invention.

A photoelectric conversion element 10a illustrated in Fig. 1 has a configuration in which a conductive film (hereinafter, also referred to as a lower electrode) 11 functioning as a lower electrode, an electron blocking film 16A, a photoelectric conversion film 12 containing the specific compound described later, and a transparent conductive film (hereinafter, also referred to as an upper electrode) 15 functioning as an upper electrode are laminated in this order.

Fig. 2 illustrates a configuration example of another photoelectric conversion element. A photoelectric conversion element 10b illustrated in Fig. 2 has a configuration in which the electron blocking film 16A, the photoelectric conversion film 12, a hole blocking film 16B, and the upper electrode 15 are laminated on the lower electrode 11 in this order. The lamination order of the electron blocking film 16A, the photoelectric conversion film 12, and the hole blocking film 16B in Figs. 1 and 2 may be appropriately changed according to the application and the characteristics.

In the photoelectric conversion element 10a (or 10b), it is preferable that light is incident on the photoelectric conversion film 12 through the upper electrode 15.

In a case where the photoelectric conversion element 10a (or 10b) is used, a voltage can be applied. In this case, it is preferable that the lower electrode 11 and the upper electrode 15 form a pair of electrodes, and a voltage of 1 × 10⁻⁵ to 1 × 10⁷ V/cm is applied between the pair of electrodes. From the viewpoint of the performance and power consumption, the applied voltage is more preferably 1 × 10⁻⁴ to 1 × 10⁷ V/cm, and still more preferably 1 × 10⁻³ to 5 × 10⁶ V/cm.

Regarding a voltage application method, in Figs. 1 and 2, it is preferable that the voltage is applied such that the electron blocking film 16A side is a cathode and the photoelectric conversion film 12 side is an anode. In a case where the photoelectric conversion element 10a (or 10b) is used as an optical sensor, or also in a case where the photoelectric conversion element 10a (or 10b) is incorporated in an imaging element, the voltage can be applied by the same method.

As described in detail below, the photoelectric conversion element 10a (or 10b) can be suitably applied to applications of the imaging element.

Hereinafter, the form of each layer constituting the photoelectric conversion element according to the embodiment of the present invention will be described in detail.

### [Photoelectric Conversion Film]

The photoelectric conversion film is a film containing a specific compound.

Hereinafter, the specific compound will be described in detail.

### <Compound (Specific Compound) Represented by Formula (1)>

The photoelectric conversion film included in the photoelectric conversion element according to the embodiment of the present invention contains the specific compound.

The specific compound is the compound represented by Formula (1).

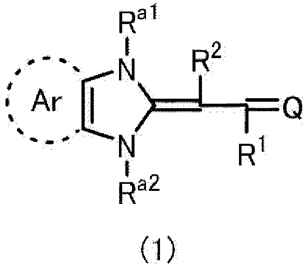

In Formula (1), R¹ and R² each independently represent a hydrogen atom or a substituent.

R¹ and R² each independently preferably represent a hydrogen atom.

In Formula (1), R^{a1} and R^{a2} each independently represent an aryl group which may have a substituent, -C(R^{L1})(R^{L2})(R^{L3}), or a heteroaryl group which may have a substituent.

The aryl group is preferably a phenyl group, a naphthyl group, or a fluorenyl group, and more preferably a phenyl group or a naphthyl group.

In a case where the aryl group is a phenyl group, the phenyl group preferably has a substituent, and the substituent is independently preferably an alkyl group (preferably having 1 to 3 carbon atoms).

In a case where the aryl group is a phenyl group, the number of substituents contained in the phenyl group is preferably 1 to 5, and more preferably 2 or 3.

In -C(R^{L1})(R^{L2})(R^{L3}), R^{L1} to R^{L3} each independently represent an alkyl group which may have a substituent, an aryl group which may have a substituent, a heteroaryl group which may have a substituent, or a hydrogen atom, and at least two of R^{L1}, R^{L2}, or R^{L3} each independently represent an alkyl group which may have a substituent, an aryl group which may have a substituent, or a heteroaryl group which may have a substituent. An alkyl group which may have a substituent, an aryl group which may have a substituent, and a heteroaryl group which may have a substituent, which are represented by R^{L1} to R^{L3}, may be bonded to each other to form a ring.

For example, the alkyl groups which may have a substituent may be bonded to each other to form a ring. A substituent in the aryl group which may have a substituent and the alkyl group which may have a substituent may be bonded to each other to form a ring. A substituent in the heteroaryl group which may have a substituent and the alkyl group which may have a substituent may be bonded to each other to form a ring. A substituent in the aryl group which may have a substituent and a substituent in another aryl group which may have a substituent may be bonded to each other to form a ring. A substituent in the aryl group which may have a substituent and a substituent in the heteroaryl group which may have a substituent may be bonded to each other to form a ring. A substituent in the heteroaryl group which may have a substituent and a substituent in another heteroaryl group which may have a substituent may be bonded to each other to form a ring.

The ring thus formed may further have a substituent, and the substituent which the formed ring has, another alkyl group which may have a substituent, a substituent in another aryl group which may have a substituent, or a substituent in another heteroaryl group which may have a substituent may be bonded to each other to further form a ring.

As described above, a group may be formed by bonding the substituent and the substituent (for example, the substituent in the aryl group which may have a substituent and the substituent in the heteroaryl group which may have a substituent) to form a single bond.

In a case where the alkyl group which may have a substituent, the aryl group which may have a substituent, and the heteroaryl group which may have a substituent, which are represented by R^{L1} to R^{L3}, may be bonded to each other to form a ring, -C(R^{L1})(R^{L2})(R^{L3}) is preferably a group other than the aryl group and the heteroaryl group.

The alkyl groups represented by R^{L1} to R^{L3} each may be independently linear, branched, or cyclic. In the alkyl groups represented by R^{L1} to R^{L3}, it is preferable that two alkyl groups are bonded to each other to form a ring.

More specifically, for example, the alkyl group represented by R^{L1} and the alkyl group represented by R^{L2} may be bonded to each other to form a ring. Furthermore, a substituent contained in a ring (a monocyclic cycloalkane ring or the like), which is formed by bonding the alkyl group represented by R^{L1} and the alkyl group represented by R^{L2} to each other, and an alkyl group represented by R^{L3} may be bonded to each other to form a polycycle (a polycyclic cycloalkane ring or the like).

That is, -C(R^{L1})(R^{L2})(R^{L3}) may be a cycloalkyl group (preferably a cyclohexyl group) which may have a substituent. The number of membered rings of the cycloalkyl group is preferably 3 to 12, more preferably 5 to 8, and still more preferably 6.

The cycloalkyl group may be monocyclic (a cyclohexyl group or the like) or polycyclic (1-adamantyl group or the like).

The cycloalkyl group preferably has a substituent. In a case where the cycloalkyl group has a substituent, a carbon atom adjacent to a carbon atom directly bonded to the nitrogen atom specified in General Formula (1) (that is, the "C" atom specified in "-C(R^{L1})(R^{L2})(R^{L3})") preferably has a substituent.

An example of a substituent which may be contained in the cycloalkyl group includes an alkyl group (preferably having 1 to 3 carbon atoms).

Substituents contained in the cycloalkyl group may be bonded to each other to form a ring, and the ring formed by bonding the substituents to each other may be a ring other than a cycloalkane ring.

R^{a1} and R^{a2} each independently preferably represent a group represented by Formula (X), -C(R^{L1})(R^{L2})(R^{L3}), a polycyclic aryl group which may have a substituent, or a polycyclic heteroaryl group which may have a substituent, from the viewpoint that the effect of the present invention is more excellent.

In particular, R^{a1} and R^{a2} are each preferably independently a group represented by Formula (X), -C(R^{L1})(R^{L2})(R^{L3}), or a polycyclic aryl group which may have a substituent, from the viewpoint that the manufacturing suitability of the photoelectric conversion element according to the embodiment of the present invention is more excellent. The group represented by Formula (X) is preferably a group represented by Formula (Z), and more preferably a group represented by Formula (ZB).

The group represented by Formula (X) is a group shown below.

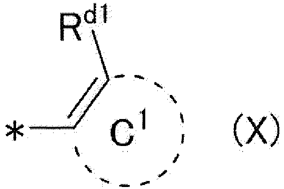

In Formula (X), C¹ represents a monocyclic aromatic ring which may have a substituent other than R^{d1}.

R^{d1} represents an alkyl group, a silyl group, an alkoxy group, an alkylthio group, a cyano group, a halogen atom, an aryl group, a heteroaryl group, an alkenyl group, or an alkynyl group.

These groups each may further have a substituent as much as possible.

Examples of the monocyclic aromatic ring include a monocyclic aromatic hydrocarbon ring and a monocyclic aromatic heterocyclic ring. An example of the aromatic hydrocarbon ring includes a benzene ring. Examples of the aromatic heterocyclic ring include a pyrrole ring, a furan ring, a thiophene ring, an imidazole ring, an oxazole ring, a thiazole ring, and other rings.

Among these, the aromatic hydrocarbon ring is preferable, and the benzene ring is more preferable, from the viewpoint that the heat resistance of the photoelectric conversion element is more excellent.

The alkyl group represented by R^{d1} preferably has 1 to 12 carbon atoms, more preferably has 1 to 6 carbon atoms, and still more preferably has 1 to 3 carbon atoms.

The silyl group represented by R^{d1} preferably has 1 to 12 carbon atoms, preferably has 1 to 6 carbon atoms, and still more preferably has 3 carbon atoms.

The alkoxy group represented by R^{d1} preferably has 1 to 12 carbon atoms, more preferably has 1 to 6 carbon atoms, and still more preferably has 1 to 3 carbon atoms.

The alkylthio group represented by R^{d1} preferably has 1 to 12 carbon atoms, more preferably has 1 to 6 carbon atoms, and still more preferably has 1 to 3 carbon atoms.

Examples of the halogen atom represented by R^{d1} include a fluorine atom, an iodine atom, a bromine atom, a chlorine atom, and the like.

The alkenyl group represented by R^{d1} preferably has 2 to 12 carbon atoms, more preferably has 2 to 6 carbon atoms, and still more preferably has 2 to 3 carbon atoms.

The alkynyl group represented by R^{d1} preferably has 2 to 12 carbon atoms, more preferably has 2 to 6 carbon atoms, and still more preferably has 2 to 3 carbon atoms.

Substituents which are contained in R^{d1} and C¹ may be bonded to each other to form a non-aromatic ring.
* represents a bonding position. The aromatic ring of C¹ is directly bonded to the nitrogen atom specified in Formula (1).

The group represented by Formula (X) is preferably the group represented by Formula (Z).

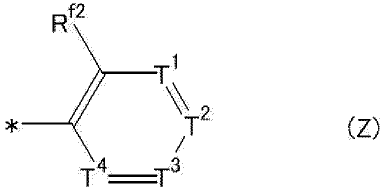

In Formula (Z), T¹ to T⁴ each independently represent -CR^{e12}= or a nitrogen atom (=N-). Re¹² represents a hydrogen atom or a substituent.

It is preferable that "at least one of T¹, T², T³, or T⁴ represents -CR^{e12}=" and at least one of Re¹² represents a substituent", and it is more preferable that "at least T⁴ represents -CR^{e12}=, and Re¹² in T⁴ represents an alkyl group, an aryl group, or a heteroaryl group".

The substituent represented by Re¹² is preferably an alkyl group, an aryl group, a heteroaryl group, a silyl group, a halogen atom, a cyano group, and the like. In addition, these groups may further have a substituent (for example, a halogen atom such as a fluorine atom).

The alkyl group represented by Re¹² preferably has 1 to 12 carbon atoms, more preferably has 1 to 6 carbon atoms, and still more preferably has 1 to 3 carbon atoms.

An example of the silyl group represented by Re¹² includes the silyl group described as the silyl group represented by R^{d1}.

Examples of the halogen atom represented by R^{e12} include a fluorine atom, an iodine atom, a bromine atom, and a chlorine atom.

In addition, in a case where a plurality of R^{e12}'s exist in Formula (Z), R^{e12}'s may be the same or different from each other.

In Formula (Z), R^{f2} represents an alkyl group, a silyl group, an alkoxy group, an alkylthio group, a cyano group, a halogen atom, an aryl group, a heteroaryl group, an alkenyl group, or an alkynyl group, and has the same meaning as R^{d1} in Formula (X) and preferable conditions are also the same.

In addition, R^{f2} and Re¹² in T¹ may be bonded to each other to form a non-aromatic ring.

The alkyl group represented by R^{f2} preferably has 1 to 12 carbon atoms, more preferably has 1 to 6 carbon atoms, and still more preferably has 1 to 3 carbon atoms.

The group represented by Formula (X) is more preferably the group represented by Formula (ZB).

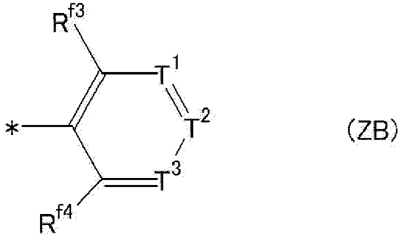

In Formula (ZB), T¹ to T³ each independently represent -CR^{e12}= or a nitrogen atom. R^{e12} represents a hydrogen atom or a substituent.

R^{e12} in Formula (ZB) is the same as Re¹² in Formula (Z).

In Formula (ZB), R^{f3} and R^{f4} each independently represent an alkyl group, an aryl group, or a heteroaryl group.

These groups each may further have a substituent as much as possible.
* represents a bonding position.

The alkyl group represented by R^{f3} or R^{f4} preferably has 1 to 12 carbon atoms, more preferably has 1 to 6 carbon atoms, and still more preferably has 1 to 3 carbon atoms.

The number of rings constituting the polycyclic aryl group which may have a substituent and the polycyclic heteroaryl group which may have a substituent is 2 or more, preferably 2 to 4, more preferably 2 to 3, and still more preferably 2.

The polycyclic aryl group which may have a substituent and a substituent which may be contained in the polycyclic heteroaryl group which may have a substituent may contain a non-aromatic ring.

As the polycyclic aryl group which may have a substituent, for example, a naphthyl group which may have a substituent is preferable.

In Formula (1), Ar represents an aromatic ring which may have a substituent.

Among these, Ar is preferably an aromatic heterocyclic ring, and more preferably a quinoxaline ring or a pyrazine ring.

The substituent which the aromatic ring represented by Ar may have is preferably an alkyl group which may have a substituent, a chlorine atom, a fluorine atom, or a cyano group.

In Formula (1), Q represents a group represented by Formula (Q1).

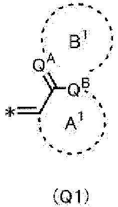

In Formula (Q1), * represents a bonding position.

That is, the compound represented by Formula (1) is the same compound as a compound represented by Formula (1a).

In addition, each symbol in Formula (1a) has the same meaning as each corresponding symbol in Formula (1) and Formula (Q1).

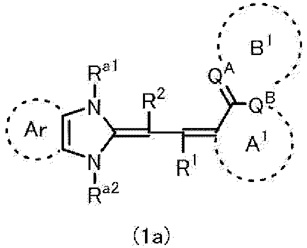

In Formula (Q1), Q^{A} represents a nitrogen atom (-N=) or -CQ^{X}=. represents a hydrogen atom or a substituent.

Among these, Q^{A} is preferably a nitrogen atom.

In Formula (Q1), Q^{B} represents a nitrogen atom (-N<), a group represented by Formula (C), or -CQ^{Y}<. Q^{Y} represents a hydrogen atom or a substituent.

Among these, Q^{B} is preferably a nitrogen atom or a group represented by Formula (C), and more preferably a nitrogen atom.

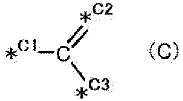

In Formula (C), *^{C1} to *^{C3} each represent a bonding position.

Among these, *^{C1} is preferably positioned at a bonding position with a carbon atom bonded to QA in Formula (Q1) by a double bond (the carbon atom is a ring member atom of both rings A1 and B1 described later).

In addition, *^{C2} is preferably a bonding position with respect to one of a ring member atom in a ring represented by A¹ described later or a ring member atom in a ring represented by B¹ described later, and *^{C3} is preferably a bonding position with respect to the other one of the ring member atom in the ring represented by A¹ described later or the ring member atom in the ring represented by B¹ described later.

In Formula (Q1), A¹ represents a ring which at least contains two carbon atoms and Q^{B}, and may have a substituent. The two carbon atoms mean carbon atoms specified in the formula.

The ring represented by A¹ may be a monocyclic ring or a polycyclic ring, may be an aromatic ring or a non-aromatic ring, or may be a ring formed with an aromatic ring and a non-aromatic ring, which are fused.

The ring represented by A¹ preferably has 5 to 15 ring member atoms, more preferably has 5 to 9 ring member atoms, and still more preferably has 5 ring member atoms.

The ring represented by A¹ may have a heteroatom (such as a nitrogen atom, an oxygen atom, and/or a sulfur atom) as a ring member atom, and preferably has 1 to 5 heteroatoms, more preferable has 1 or 2 heteroatoms, and still more preferably has 2 heteroatoms. The number of heteroatoms described herein is a number including the number of nitrogen atoms that may be represented by Q^{B}.

Among these, the ring represented by A¹ is preferably a ring containing a linking group represented by Formula (AA) or (AB), and is more preferably a ring containing a linking group represented by Formula (AA).

*^{V}-CR^{Q1}=N-*^{W} ... (AA)

*^{V}-Arylene group-*^{W} ... (AB)

In each of Formulae (AA) and (AB), *^{V} represents a bonding position to the root carbon atom in Formula (Q1). The root carbon atom means a carbon atom in Formula (Q1) that is bonded to by a double bond a carbon atom that is bonded to R¹ in Formula (1).

In each of Formulae (AA) and (AB), *^{W} represents a bonding position to Q^{B}.

In Formula (AA), R^{Q1} represents a hydrogen atom or a substituent. A substituent represented by R^{Q1} is preferably a fluorine atom, a chlorine atom, a cyano group, an alkyl group which may have a substituent, an aryl group which may have a substituent, or a heteroaryl group which may have a substituent.

The arylene group in Formula (AB) is preferably a group obtained by removing a hydrogen atom from each of two adjacent ring member atoms in an aromatic hydrocarbon ring. The arylene group may have a substituent. The arylene group is preferably a 1,2-phenylene group.

In Formula (Q2), B¹ represents a ring which at least contains one carbon atom, Q^{A}, and Q^{B}, and may have a substituent.

The ring represented by B¹ may be a monocyclic ring or a polycyclic ring, may be an aromatic ring or a non-aromatic ring, or may be a ring formed with an aromatic ring and a non-aromatic ring, which are fused.

The ring represented by B¹ preferably has 5 to 15 ring member atoms, more preferably has 5 to 9 ring member atoms, and still more preferably has 5 to 6 ring member atoms.

The ring represented by B¹ may have a heteroatom (such as a nitrogen atom, an oxygen atom, and/or a sulfur atom) as a ring member atom, and preferably has 1 to 5 heteroatoms, and more preferable has 2 or 3 heteroatoms. The number of heteroatoms described herein is a number including the number of nitrogen atoms that may be represented by Q^{A} or Q^{B}.

Among these, the ring represented by B¹ is preferably a ring containing a linking group represented by Formula (BA) or (BB).

Here, in a case where the linking group is a linking group represented by Formula (BA), Q^{B} is a nitrogen atom or -CQ^{Y}<.

In a case where the linking group is a linking group represented by Formula (BB), Q^{B} is the group represented by Formula (C).

*^{X}-(CO)ₘ-T-(CO)ₙ-*^{Y} ... (BA)

*^{X}-X=Y-Z=*^{Y} ... (BB)

In Formulae (BA) and (BB), *^{X} represents a bonding position to Q^{A}.

In Formulae (BA) and (BB), *^{Y} represents a bonding position to Q^{B}.

In Formula (BA), m and n each independently represent 0 or 1.

In Formula (BA), T represents -CR^{Q2}=CR^{Q2}-, -CR^{Q2}=N-, -N=N-, -NR^{Q2}-, -O-, or -C(R^{Q2})₂-. R^{Q2} represents a hydrogen atom or a substituent. A substituent represented by R^{Q2} is preferably a cyano group, an alkyl group which may have a substituent, an aryl group which may have a substituent, or a heteroaryl group which may have a substituent. Two R^{Q2}'s in -CR^{Q2}=CR^{Q2}- may be bonded to each other to form a ring (for example, an aromatic ring).

The nitrogen atom (=N-) at -CR^{Q2}=N- may be present on the *^{X} side or *^{Y} side.

In Formula (BB), X, Y, and Z each independently represent -CR^{Q3}= or -N=, and -CR^{Q3}= is preferable. R^{Q3} represents a hydrogen atom or a substituent. A substituent represented by R^{Q3} is preferably a fluorine atom, a chlorine atom, a cyano group, an alkyl group which may have a substituent, an aryl group which may have a substituent, or a heteroaryl group which may have a substituent.

Q in Formula (1) preferably represents a group represented by Formula (Q2).

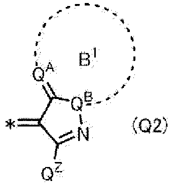

In Formula (Q2), * represents a bonding position.

In Formula (Q2), Q^{A} represents a nitrogen atom or -CQ^{X}=. Q^{X} represents a hydrogen atom or a substituent.

In Formula (Q2), Q^{B} represents a nitrogen atom, a group represented by Formula (C), or -CQ^{Y}<. Q^{Y} represents a hydrogen atom or a substituent.

The group represented by Formula (C) is as described above. In addition, in the group represented by Formula (Q2), in a case where Q^{B} is the group represented by Formula (C), *^{C2} in Formula (C) is a bonding position to a ring member atom in the ring represented by B1.

In Formula (Q2), B¹ represents a ring which at least contains one carbon atom, Q^{A}, and Q^{B}, and may have a substituent. The above-described one carbon atom means a carbon atom sandwiched between Q^{A} and Q^{B}, which are specified in Formula (Q2).

Q^{A}, Q^{B}, and B¹ in Formula (Q2) have the same meaning as Q^{A}, Q^{B}, and B¹ in Formula (Q1), respectively, and preferable conditions are also the same.

In Formula (Q2), Q^{Z} represents a hydrogen atom or a substituent.

A substituent represented by Q^{Z} is preferably a fluorine atom, a chlorine atom, a cyano group, an alkyl group which may have a substituent, an aryl group which may have a substituent, or a heteroaryl group which may have a substituent.

Q in Formula (1) more preferably represents a group represented by Formula (Q3).

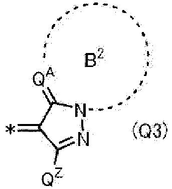

In Formula (Q3), * represents a bonding position.

In Formula (Q3), Q^{A} represents a nitrogen atom or -CQ^{X}=. Q^{X} represents a hydrogen atom or a substituent.

In Formula (Q3), Q^{Z} represents a hydrogen atom or a substituent.

Q^{A} in Formula (Q3) has the same meaning as Q^{A} in Formula (Q1) and preferable conditions are also the same.

Q^{Z} in Formula (Q3) has the same meaning as Q^{Z} in Formula (Q2) and preferable conditions are also the same.

In Formula (Q3), B² represents a ring which at least contains one carbon atom, one nitrogen atom, and Q^{A}, and may have a substituent. The above-described one carbon atom means a carbon atom sandwiched between Q^{A} and a nitrogen atom, which are specified in Formula (Q3). The above-described one nitrogen atom means a nitrogen atom constituting the ring specified in Formula (Q3) and represented by B².

The ring represented by B² may be a monocyclic ring or a polycyclic ring, may be an aromatic ring or a non-aromatic ring, or may be a ring formed with an aromatic ring and a non-aromatic ring, which are fused.

The ring represented by B² preferably has 5 to 15 ring member atoms, more preferably has 5 to 9 ring member atoms, and still more preferably has 5 to 6 ring member atoms.

The ring represented by B² preferably has 1 to 5 heteroatoms (such as nitrogen atoms, oxygen atoms, and/or sulfur atoms) as a ring member atom, and more preferably has 2 or 3 heteroatoms. The number of heteroatoms described herein is a number including the number of nitrogen atoms that are specified in Formula (Q3) and that may be represented by Q^{A}.

Among these, the ring represented by B² in Formula (Q3) is preferably a ring containing a linking group represented by Formula (BA). Here, in this case, *^{Y} in Formula (BA) is a bonding position to a nitrogen atom. The nitrogen atom bonded by *^{Y} is a nitrogen atom having a form of Q^{B} in Formula (Q1).

The specific compound is preferably a compound represented by Formula (2).

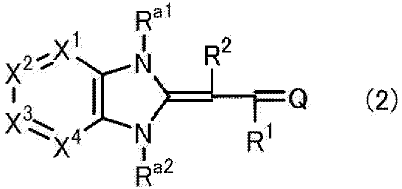

In Formula (2), R¹ and R² each independently represent a hydrogen atom or a substituent.

In Formula (2), R^{a1} and R^{a2} each independently represent an aryl group which may have a substituent, -C(R^{L1})(R^{L2})(R^{L3}), or a heteroaryl group which may have a substituent.

In Formula (2), Q represents a group represented by Formula (Q1).

R¹, R², R^{a1}, R^{a2}, and Q in Formula (2) have the same meanings as R¹, R², R^{a1}, R^{a2}, and Q in Formula (1), respectively, and preferable conditions are also the same.

In Formula (2), X¹ to X⁴ each independently represent a nitrogen atom (-N=) or -CR^{c1}=.

R^{c1} represents a hydrogen atom or a substituent.

In a case where a plurality of R^{c1}'s exist, the plurality of R^{c1}'s may be bonded to each other to form a ring.

For example, X¹ and X² may be both -CR^{c1}=, and these R^{c1}'s may be bonded to each other to form a ring. X² and X³ may be both -CR^{c1}=, and these R^{c1}'s may be bonded to each other to form a ring. X³ and X⁴ may be both -CR^{c1}=, and these R^{c1}'s may be bonded to each other to form a ring.

The ring formed by bonding the plurality of R^{c1}'s to each other may be an aromatic ring (benzene ring or another ring) or a non-aromatic ring, may have one or more (for example, 1 to 6) heteroatoms, and may further have a substituent. A plurality of substituents in the ring formed by bonding R^{c1}'s to each other may be bonded to each other to further form a ring (a ring which may have a substituent).

The specific compound is more preferably a compound represented by Formula (3).

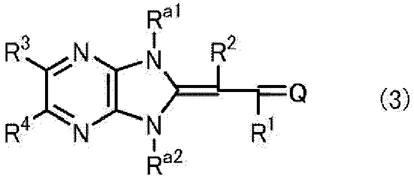

In Formula (3), R¹ and R² each independently represent a hydrogen atom or a substituent.

In Formula (3), R^{a1} and R^{a2} each independently represent an aryl group which may have a substituent, -C(R^{L1})(R^{L2})(R^{L3}), or a heteroaryl group which may have a substituent.

In Formula (3), Q represents a group represented by Formula (Q1).

R¹, R², R^{a1}, R^{a2}, and Q in Formula (3) have the same meanings as R¹, R², R^{a1}, R^{a2}, and Q in Formula (1), respectively, and preferable conditions are also the same.

In Formula (3), R³ and R⁴ each independently represent a hydrogen atom or a substituent.

R³ and R⁴ may be bonded to each other to form a ring.

The ring formed by bonding R³ and R⁴ to each other may be an aromatic ring or a non-aromatic ring, may have one or more (for example, 1 to 6) heteroatoms, and may further have a substituent. A plurality of substituents in the ring formed by bonding R³ and R⁴ to each other may be bonded to each other to further form a ring (a ring which may have a substituent).

The specific compound is still more preferably a compound represented by Formula (4).

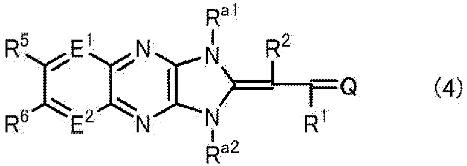

In Formula (4), R¹ and R² each independently represent a hydrogen atom or a substituent.

In Formula (4), R^{a1} and R^{a2} each independently represent an aryl group which may have a substituent, -C(R^{L1})(R^{L2})(R^{L3}), or a heteroaryl group which may have a substituent.

In Formula (4), Q represents a group represented by Formula (Q1).

R¹, R², R^{a1}, R^{a2}, and Q in Formula (4) have the same meanings as R¹, R², R^{a1}, R^{a2}, and Q in Formula (1), respectively, and preferable conditions are also the same.

In Formula (4), E¹ represents a nitrogen atom or -CR^{E1}=. R^{E1} represents a hydrogen atom or a substituent.

In Formula (4), E² represents a nitrogen atom or -CR^{E2}=. R^{E2} represents a hydrogen atom or a substituent.

R^{E1} serving as a substituent and R^{E2} serving as a substituent are each independently preferably an alkoxy group, a silyl group, a chlorine atom, a fluorine atom, a cyano group, or an alkyl group, and more preferably an alkoxy group containing an alkyl group moiety having 1 to 3 carbon atoms, a chlorine atom, a fluorine atom, a cyano group, or an alkyl group having 1 to 4 carbon atoms.

In Formula (4), R⁵ and R⁶ each independently represent a hydrogen atom or a substituent.

R⁵ serving as a substituent and R⁶ serving as a substituent are each independently preferably an alkoxy group, a silyl group, a chlorine atom, a fluorine atom, a cyano group, or an alkyl group, and more preferably an alkoxy group containing an alkyl group moiety having 1 to 3 carbon atoms, a chlorine atom, a fluorine atom, a cyano group, or an alkyl group having 1 to 4 carbon atoms.

R⁵ and R⁶ may be bonded to each other to form a ring.

The ring formed by bonding R⁵ and R⁶ to each other may be an aromatic ring (benzene ring or another ring) or a non-aromatic ring, may have one or more (for example, 1 to 6) heteroatoms, and may further have a substituent. A plurality of substituents in the ring formed by bonding R⁵ and R⁶ to each other may be bonded to each other to further form a ring (a ring which may have a substituent).

The specific compounds are exemplified below.

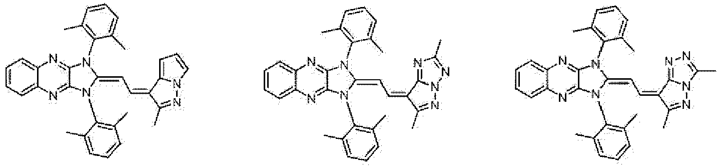

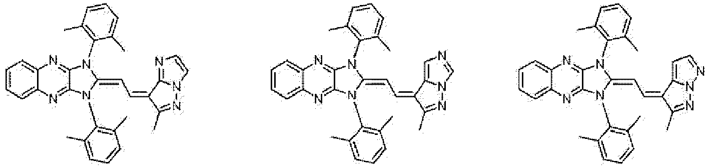

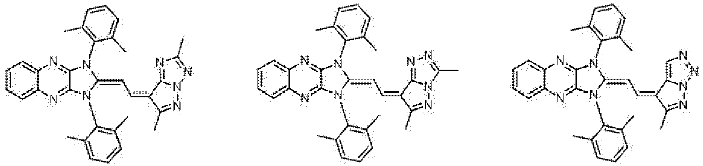

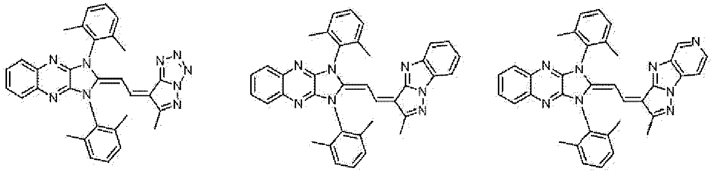

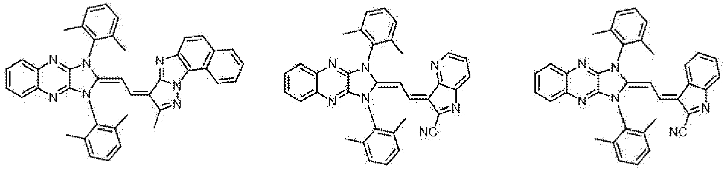

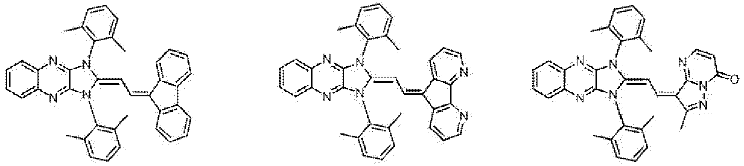

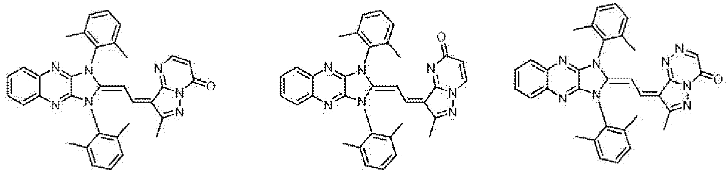

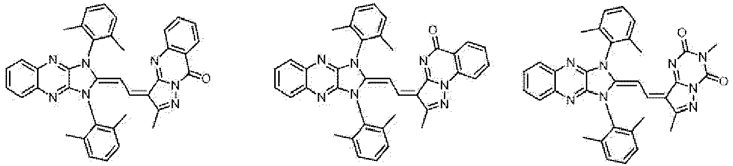

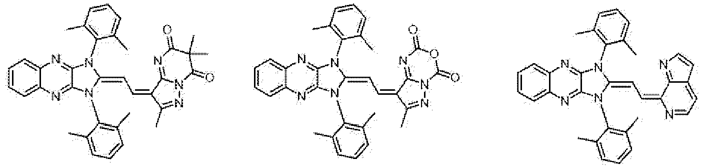

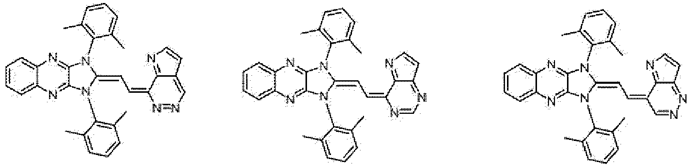

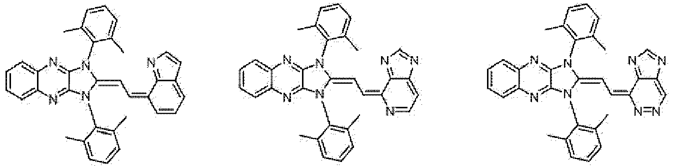

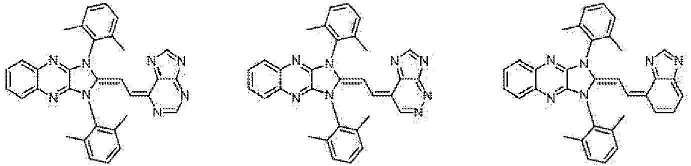

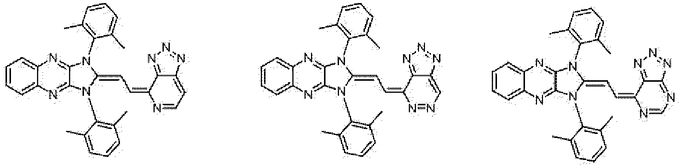

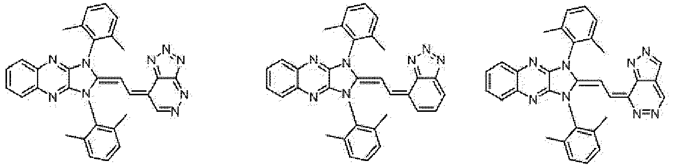

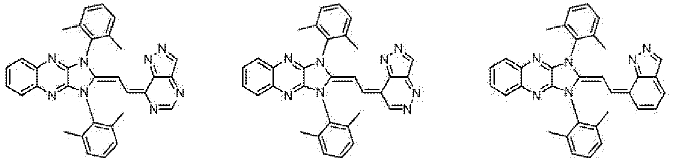

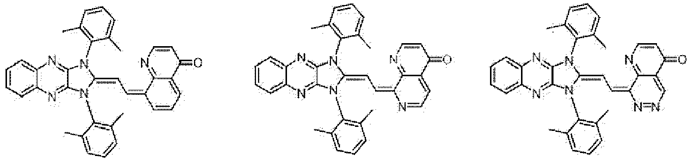

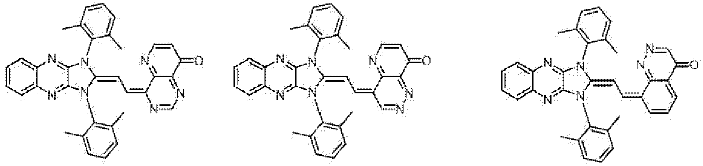

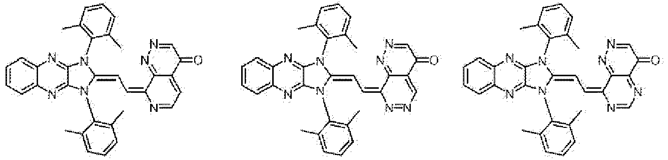

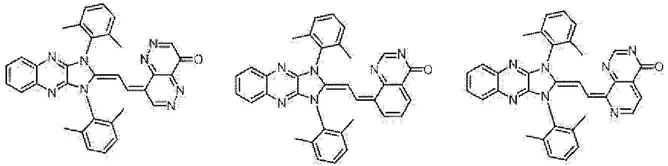

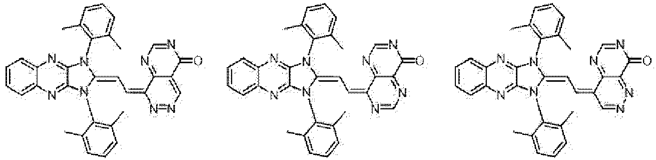

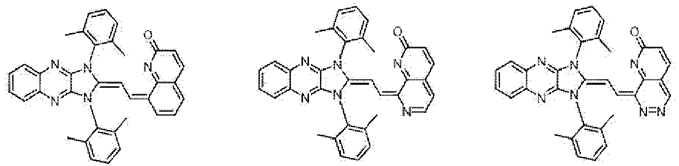

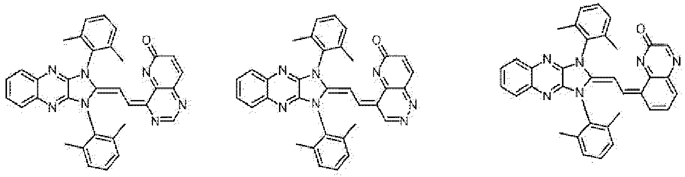

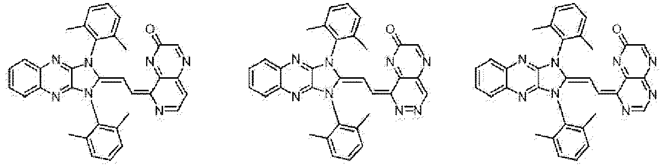

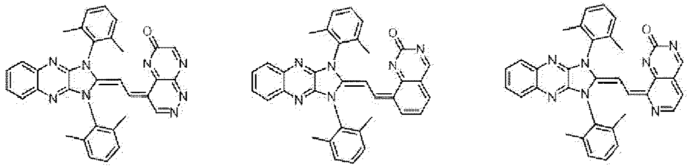

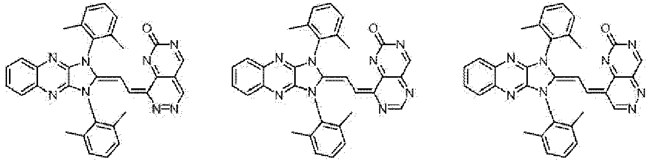

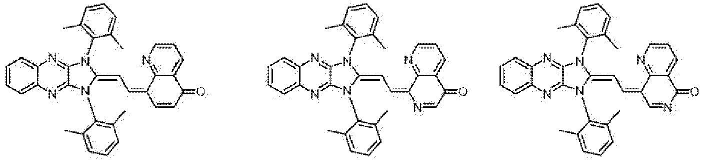

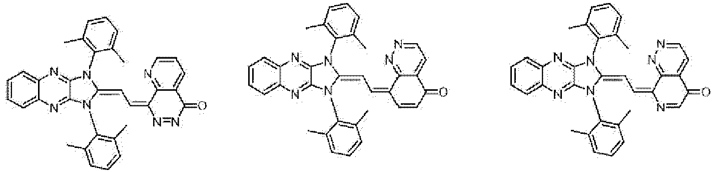

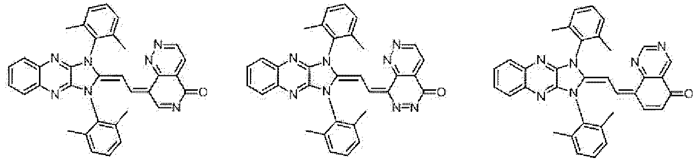

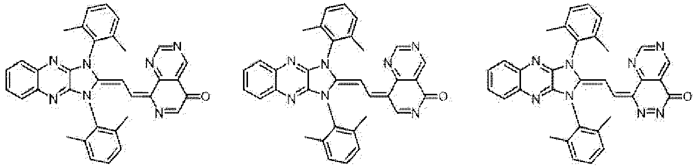

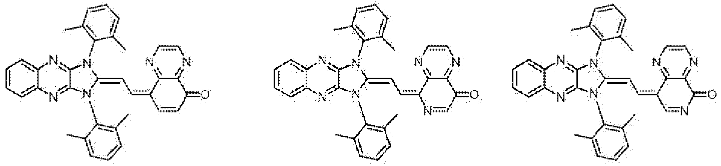

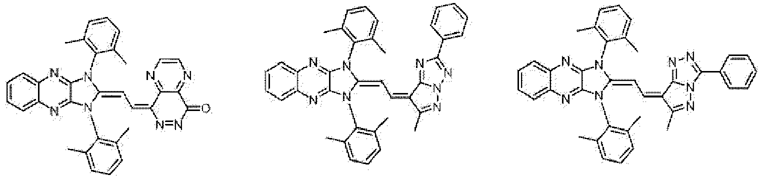

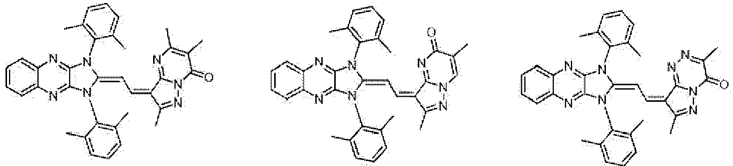

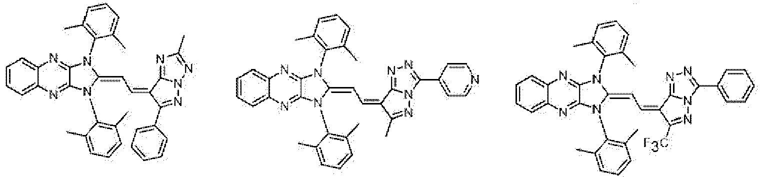

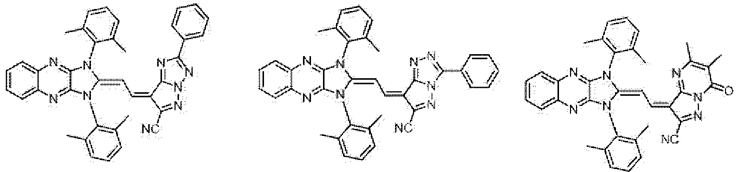

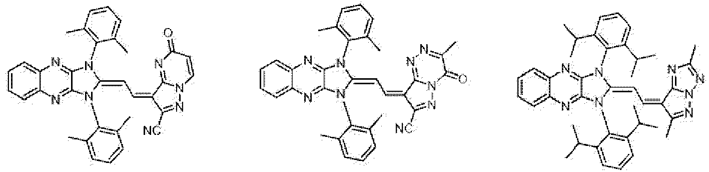

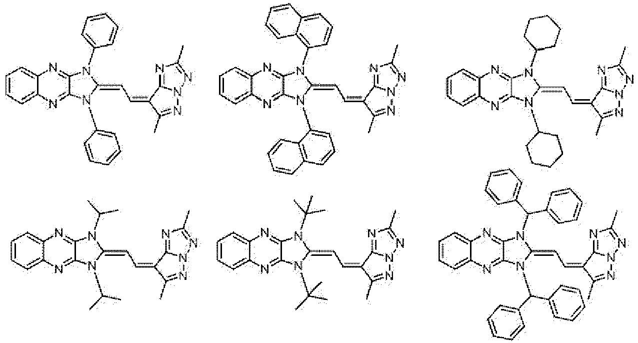

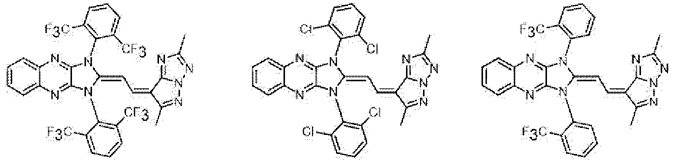

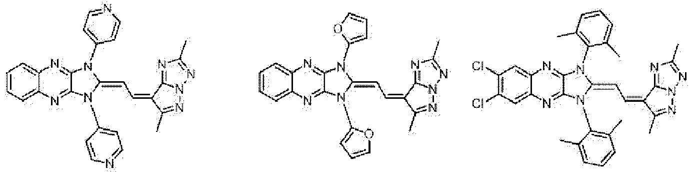

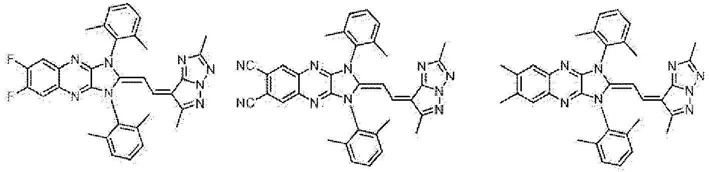

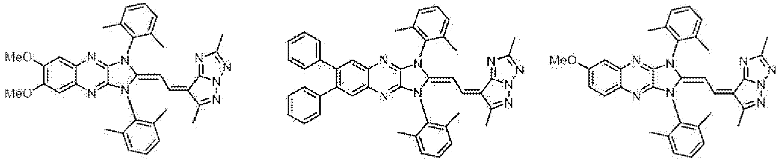

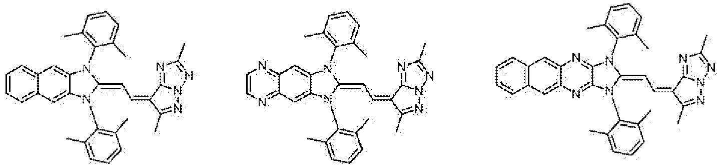

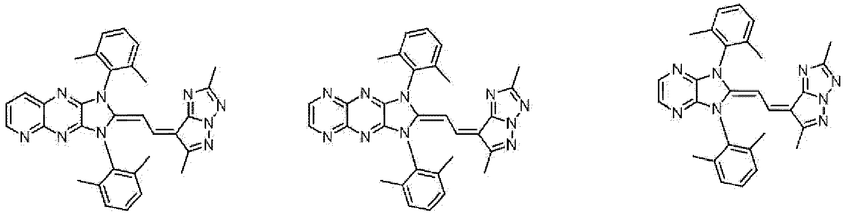

A molecular weight of the specific compound is not particularly limited, but is preferably 400 to 1200. In a case where the molecular weight is 1200 or less, a vapor deposition temperature is not increased, and the compound is not easily decomposed. In a case where the molecular weight is 400 or more, a glass transition point of a vapor deposition film is not lowered, and the heat resistance of the photoelectric conversion element is improved.

The specific compound is particularly useful as a material of the photoelectric conversion film used for the imaging element, the optical sensor, or a photoelectric cell. In addition, the specific compound usually functions as the p-type organic semiconductor in the photoelectric conversion film in many cases. The specific compound can also be used as a coloring material, a liquid crystal material, an organic semiconductor material, a charge transport material, a pharmaceutical material, and a fluorescent diagnostic material.

The specific compound is preferably a compound in which an ionization potential in a single film is -5.0 to -6.0 eV from the viewpoints of stability in a case of using the compound as the p-type organic semiconductor and matching of energy levels between the compound and the n-type organic semiconductor.

The maximum absorption wavelength of the specific compound is not particularly limited, but is preferably within a range of 500 to 600 nm, and more preferably within a range of 530 to 580 nm.

The maximum absorption wavelength is a value measured in a film state of the specific compound (for example, a vapor deposition film of the specific compound).

The specific compound may be purified as necessary.

A purification method of the specific compound is not particularly limited, but sublimation purification is preferably employed.

The purity of the specific compound after sublimation purification (for example, the purity measured by HPLC or GC) is not particularly limited, but is preferably 95% or more, more preferably 98% or more, and still more preferably 99% or more.

Before the specific compound is sublimated and purified, the specific compound may be purified by another method. For example, the specific compound is preferably purified by using silica gel column chromatography, the GEL PERMEATION CHROMATOGRAPHY (GPC), reslurry washing, reprecipitation purification, purification with an adsorbent such as activated carbon, or recrystallization purification.

The purity of the specific compound before sublimation purification (for example, the purity measured by HPLC or GC) is not particularly limited, but is preferably 95% or more, more preferably 98% or more, and still more preferably 99% or more.

A solvent (recrystallizing solvent) used in the recrystallization purification is not particularly limited, and examples thereof include methanol, ethanol, isopropanol, butanol, toluene, xylene, anisole, 1,2-dimethoxybenzene, tetralin, chlorobenzene, dichlorobenzene, hexane, heptane, octane, acetonitrile, benzonitrile, acetic acid, chloroform, dichloromethane, ethyl acetate, butyl acetate, tetrahydrofuran, 4-methyltetrahydropyran, cyclopentylmethyl ether, and other solvents.

The recrystallizing solvent may be a mixed solution in which a plurality of solvents are mixed.

The amount of a residual solvent contained in a crude product containing the specific compound to be subjected to the sublimation purification is not particularly limited, but the amount of the residual solvent is preferably 10% by mol or less, more preferably 5% by mol or less, and still more preferably 2% by mol or less with respect to the total molar amount of the specific compound in the crude product.

Impurities including elements (for example, Li, Na, K, Mg, Ca, Al, Si, P, Sn, transition metal elements, or other elements) that do not constitute the specific compound contained in the crude product containing the specific compound to be subjected to the sublimation purification are not particularly limited, but the impurities are preferably 1000 mass ppm or less, more preferably 100 mass ppm or less, and still more preferably 10 mass ppm or less with respect to the total mass of the crude product.

Examples of the method for measuring the elements include an ICP (high frequency inductively coupled plasma) emission analysis method.

The specific compound can be synthesized by a known method.

The purity of raw materials used to synthesize the specific compound (for example, the purity measured by HPLC or GC), including intermediates, to improve the purity of the specific compound is not particularly limited, but is preferably 97% or more, more preferably 98% or more, and still more preferably 99% or more.

In a case where the purity of the commercially available raw materials or synthetic intermediates is low, those purified by a known method may be used.

A content of the specific compound in the photoelectric conversion film (=film thickness of specific compound in terms of single layer/film thickness of photoelectric conversion film × 100) is preferably 15% to 75% by volume, more preferably 20% to 60% by volume, and still more preferably 25% to 50% by volume.

The specific compound may be used alone, or two or more thereof may be used in combination.

### <n-Type Organic Semiconductor>

It is preferable that the photoelectric conversion film contains the n-type organic semiconductor as a component other than the specific compound.

The n-type organic semiconductor is an acceptor-property organic semiconductor material (a compound), and refers to an organic compound having a property of easily accepting an electron. More specifically, the n-type organic semiconductor refers to an organic compound having a large electron affinity of two organic compounds used in contact with each other. Therefore, any organic compound having an electron accepting property can be used as the acceptor type organic semiconductor.

Examples of the n-type organic semiconductor include fullerenes selected from the group consisting of a fullerene and derivatives thereof, fused aromatic carbocyclic compounds (for example, a naphthalene derivative, an anthracene derivative, a phenanthrene derivative, a tetracene derivative, a pyrene derivative, a perylene derivative, and a fluoranthene derivative); a heterocyclic compound having a 5- to 7-membered ring having at least one of a nitrogen atom, an oxygen atom, or a sulfur atom (for example, pyridine, pyrazine, pyrimidine, pyridazine, triazine, quinoline, quinoxaline, quinazoline, phthalazine, cinnoline, isoquinoline, pteridine, acridine, phenazine, phenanthroline, tetrazole, pyrazole, imidazole, and thiazole); polyarylene compounds; fluorene compounds; cyclopentadiene compounds; silyl compounds; 1,4,5,8-naphthalenetetracarboxylic acid anhydride; 1,4,5,8-naphthalenetetracarboxylic acid anhydride imide derivative; oxadiazole derivative; anthraquinodimethane derivatives; diphenylquinone derivatives; bathocuproine, bathophenanthroline, and derivatives thereof; triazole compounds; a distyrylarylene derivative; a metal complex having a nitrogen-containing heterocyclic compound as a ligand; a silole compound; and compounds disclosed in paragraphs [0056] to [0057] of JP2006-100767A.

Among these, it is preferable that examples of the n-type organic semiconductor (compound) include fullerenes selected from the group consisting of a fullerene and derivatives thereof.

Examples of the fullerenes include a fullerene C60, a fullerene C70, a fullerene C76, a fullerene C78, a fullerene C80, a fullerene C82, a fullerene C84, a fullerene C90, a fullerene C96, a fullerene C240, a fullerene C540, and a mixed fullerene.

Examples of the fullerene derivatives include compounds in which a substituent is added to the above fullerenes. The substituent is preferably an alkyl group, an aryl group, or a heterocyclic group. The fullerene derivative is preferably compounds described in JP2007-123707A.

An organic coloring agent may be used as the n-type organic semiconductor. Examples of the organic coloring agent include a cyanine coloring agent, a styryl coloring agent, a hemicyanine coloring agent, a merocyanine coloring agent (including zeromethine merocyanine (simple merocyanine)), a rhodacyanine coloring agent, an allopolar coloring agent, an oxonol coloring agent, a hemioxonol coloring agent, a squarylium coloring agent, a croconium coloring agent, an azamethine coloring agent, a coumarin coloring agent, an arylidene coloring agent, an anthraquinone coloring agent, a triphenylmethane coloring agent, an azo coloring agent, an azomethine coloring agent, a metallocene coloring agent, a fluorenone coloring agent, a flugide coloring agent, a perylene coloring agent, a phenazine coloring agent, a phenothiazine coloring agent, a quinone coloring agent, a diphenylmethane coloring agent, a polyene coloring agent, an acridine coloring agent, an acridinone coloring agent, a diphenylamine coloring agent, a quinophthalone coloring agent, a phenoxazine coloring agent, a phthaloperylene coloring agent, a dioxane coloring agent, a porphyrin coloring agent, a chlorophyll coloring agent, a phthalocyanine coloring agent, a subphthalocyanine coloring agent, and a metal complex coloring agent.

The molecular weight of the n-type organic semiconductor is preferably 200 to 1200, and more preferably 200 to 900.

It is also desirable that the n-type organic semiconductor is colorless or has an absorption maximum wavelength and/or an absorption waveform close to that of the specific compound. As specific numerical values, it is preferable that the absorption maximum wavelength of the n-type organic semiconductor is in a range of 400 nm or less or 500 to 600 nm.

It is preferable that the photoelectric conversion film has a bulk hetero structure formed in a state in which the specific compound and the n-type organic semiconductor are mixed. The bulk hetero structure refers to a layer in which the specific compound and the n-type organic semiconductor are mixed and dispersed in the photoelectric conversion film. The photoelectric conversion film having the bulk hetero structure can be formed by either a wet method or a dry method. The bulk hetero structure is described in detail in, for example, paragraphs [0013] and [0014] of JP2005-303266A and the like.

In a case where the photoelectric conversion film contains the n-type organic semiconductor, a content of the n-type organic semiconductor in the photoelectric conversion film (=film thickness of n-type organic semiconductor in terms of single layer/film thickness of photoelectric conversion film × 100) is preferably 15% to 75% by volume, more preferably 20% to 60% by volume, and still more preferably 25% to 50% by volume.

The n-type semiconductor material may be used alone, or two or more thereof may be used in combination.

In addition, in a case where the n-type semiconductor material includes fullerenes, a content of the fullerenes to a total content of the n-type semiconductor material (= film thickness of fullerenes in terms of single layer/total film thickness of n-type semiconductor materials in terms of single layer × 100) is preferably 50% to 100% by volume, and more preferably 80% to 100% by volume.

The fullerenes may be used alone, or two or more thereof may be used in combination.

The difference in electron affinity between the specific compound and the n-type organic semiconductor is preferably 0.1 eV or more.

From the viewpoint of responsiveness of the photoelectric conversion element, the content of the specific compound to the total content of the specific compound and the n-type organic semiconductor (= film thickness in terms of single layer of specific compound/(film thickness in terms of single layer of specific compound + film thickness in terms of single layer of n-type organic semiconductor) × 100) is preferably 20% to 80% by volume, and more preferably 40% to 80% by volume.

Also, in a case where the photoelectric conversion film contains a p-type organic semiconductor described below, the content of the specific compound (= film thickness in terms of single layer of specific compound/(film thickness in terms of single layer of specific compound + film thickness in terms of single layer of n-type organic semiconductor + film thickness in terms of single layer of p-type organic semiconductor) × 100) is preferably 15% to 75% by volume, and more preferably 30% to 75% by volume.

It is preferable that the photoelectric conversion film is substantially formed of the specific compound, the n-type organic semiconductor, and the p-type organic semiconductor included as desired. The term "substantially" means that a total content of the specific compound, the n-type organic semiconductor, and the p-type organic semiconductor included as desired is 90% to 100% by volume (preferably 95% to 100% by volume, and more preferably 99% to 100% by volume) with respect to a total mass of the photoelectric conversion film.

### <p-Type Organic Semiconductor>

In addition, the photoelectric conversion film may include a p-type organic semiconductor.

In particular, the photoelectric conversion film preferably further contains the p-type organic semiconductor in addition to the specific compound and the n-type organic semiconductor.

Examples of the p-type organic semiconductor include the following compounds.

The p-type organic semiconductor here means a p-type organic semiconductor which is a compound different from the specific compound.

The p-type organic semiconductor is a donor organic semiconductor material (a compound), and refers to an organic compound having a property of easily donating an electron. More specifically, the p-type organic semiconductor means an organic compound having a smaller ionization potential in a case where two organic compounds are used in contact with each other.

Examples of the p-type organic semiconductor include triarylamine compounds (for example, N, N'-bis (3-methylphenyl)-(1,1'-biphenyl)-4,4'-diamine (TPD), 4,4'-bis [N-(naphthyl)-N-Phenyl-amino] biphenyl (α-NPD), compounds disclosed in paragraphs [0128] to [0148] of JP2011-228614A, compounds disclosed in paragraphs [0052] to [0063] of JP2011-176259A, compounds disclosed in paragraphs [0119] to [0158] of JP2011-225544A, compounds disclosed in paragraphs [0044] to [0051] of JP2015-153910A, and compounds disclosed in paragraphs [0086] to [0090] of JP2012-094660A), pyrazoline compounds, styrylamine compounds, hydrazone compounds, polysilane compounds, thiophene compounds (for example, a thienothiophene derivative, a dibenzothiophene derivative, a benzodithiophene derivative, a dithienothiophene derivative, a [1]benzothieno[3,2-b]thiophene (BTBT) derivative, a thieno [3,2-f: 4,5-f] bis [1] benzothiophene (TBBT) derivative, compounds disclosed in paragraphs [0031] to [0036] of JP2018-014474A, compounds disclosed in paragraphs [0043] to [0045] of WO2016-194630A, compounds disclosed in paragraphs [0025] to [0037], and [0099] to [0109] of WO2017-159684A, compounds disclosed in paragraphs [0029] to [0034] of JP2017-076766A, compounds disclosed in paragraphs [0015] to [0025] of WO2018-207722A, compounds disclosed in paragraphs [0045] to [0053] of JP2019-054228A, compounds disclosed in paragraphs [0045] to [0055] of WO2019-058995A, compounds disclosed in paragraphs [0063] to [0089] of WO2019-081416A, compounds disclosed in paragraphs [0033] to [0036] of JP2019-080052A, compounds disclosed in paragraphs [0044] to [0054] of WO2019-054125A, compounds disclosed in paragraphs [0041] to [0046] of WO2019-093188A, compounds in paragraphs [0034] to [0037] of JP2019-050398A, compounds in paragraphs [0033] to [0036] of JP2018-206878A, compounds in paragraph [0038] of JP2018-190755A, compounds in paragraphs [0019] to [0021] of JP2018-026559A, compounds in paragraphs [0031] to [0056] of JP2018-170487A, compounds in paragraphs [0036] to [0041] of JP2018-078270A, compounds in paragraphs [0055] to [0082] of JP2018-166200A, compounds in paragraphs [0041] to [0050] of JP2018-113425A, compounds in paragraphs [0044] to [0048] of JP2018-85430A, compounds in paragraphs [0041] to [0045] of JP2018-056546A, compounds in paragraphs [0042] to [0049] of JP2018-046267A, compounds in paragraphs [0031] to [0036] of JP2018-014474A, compounds disclosed in paragraphs [0036] to [0046] of WO2018-016465A, compounds disclosed in paragraphs [0045] to [0048] of JP2020-010024A, and the like), a cyanine compound, an oxonol compound, a polyamine compound, an indole compound, a pyrrole compound, a pyrazole compound, a polyarylene compound, a fused aromatic carbocyclic compound (for example, a naphthalene derivative, an anthracene derivative, a phenanthrene derivative, a tetracene derivative, a pentacene derivative, a pyrene derivative, a perylene derivative, and a fluoranthene derivative), a porphyrin compound, a phthalocyanine compound, a triazole compound, an oxadiazole compound, an imidazole compound, a polyarylalkane compound, a pyrazolone compound, an amino-substituted chalcone compound, an oxazole compound, a fluorenone compound, a silazane compound, and a metal complex having nitrogen-containing heterocyclic compounds as ligands.

Examples of the p-type organic semiconductor include compounds having an ionization potential smaller than that of the n-type organic semiconductor, and in a case where this condition is satisfied, the organic coloring agents exemplified as the n-type organic semiconductor can be used.

The compounds that can be used as the p-type semiconductor compound are exemplified below.

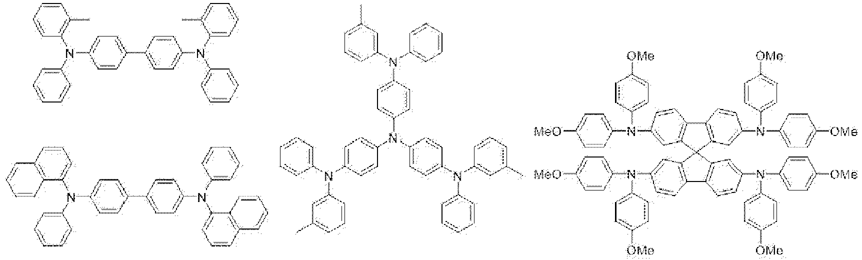

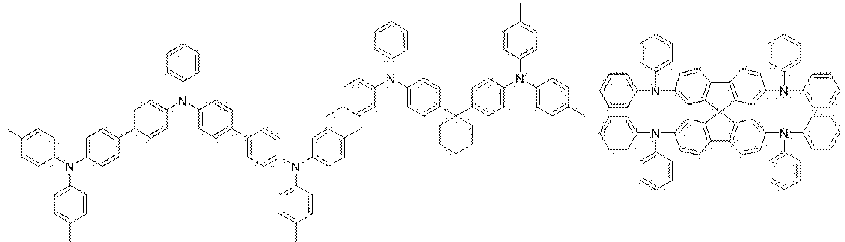

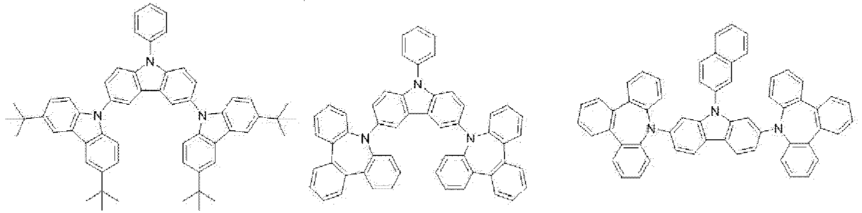

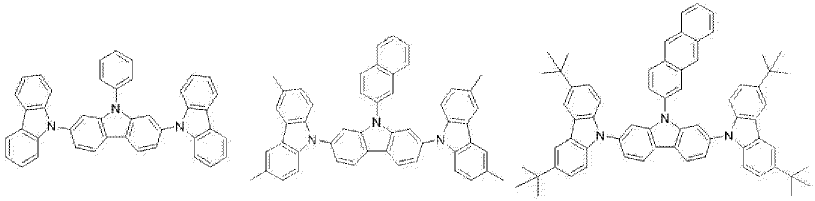

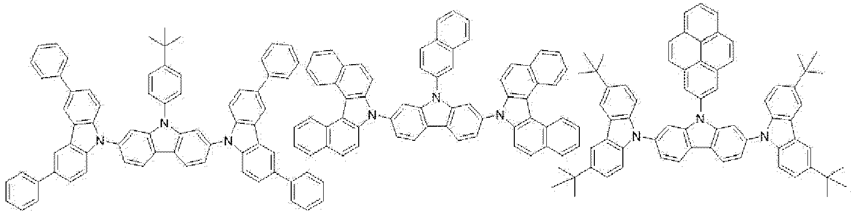

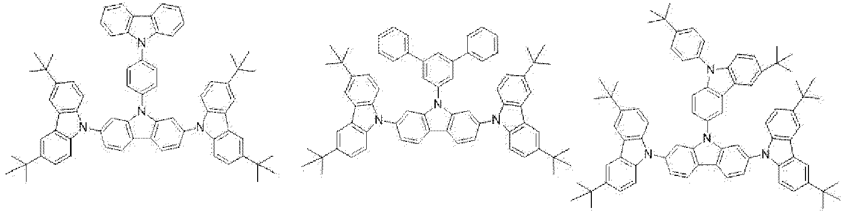

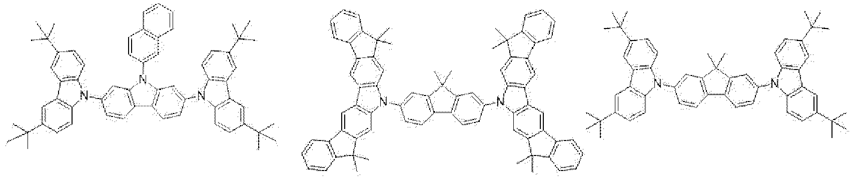

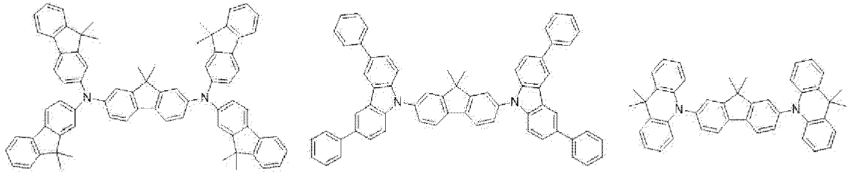

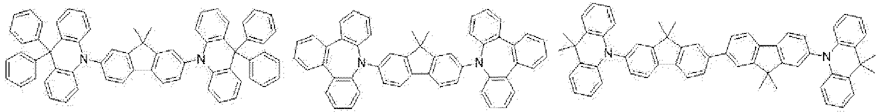

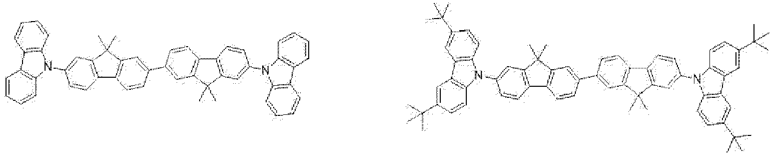

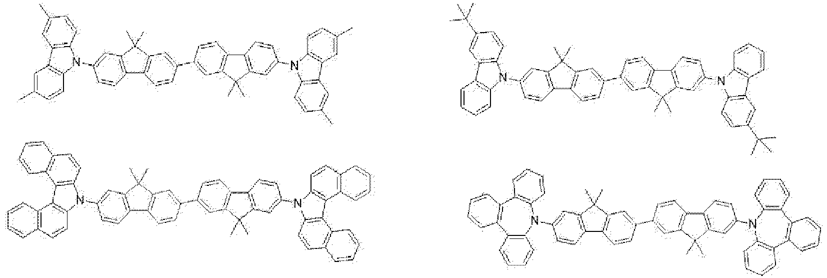

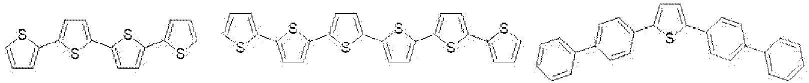

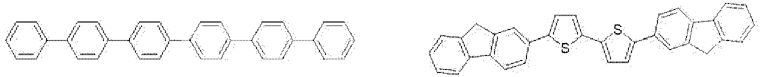

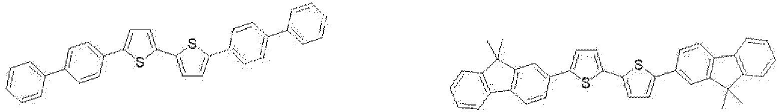

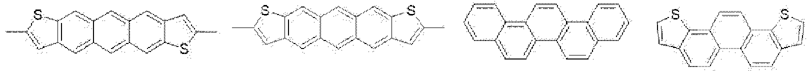

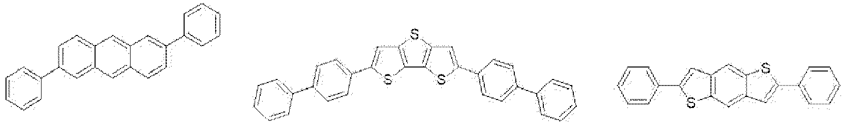

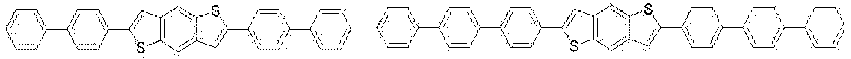

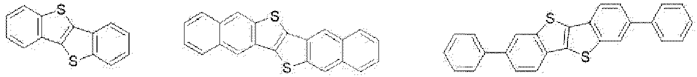

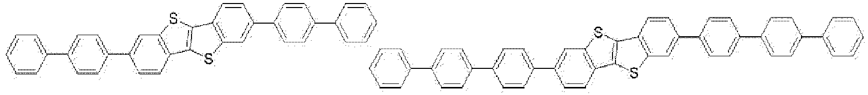

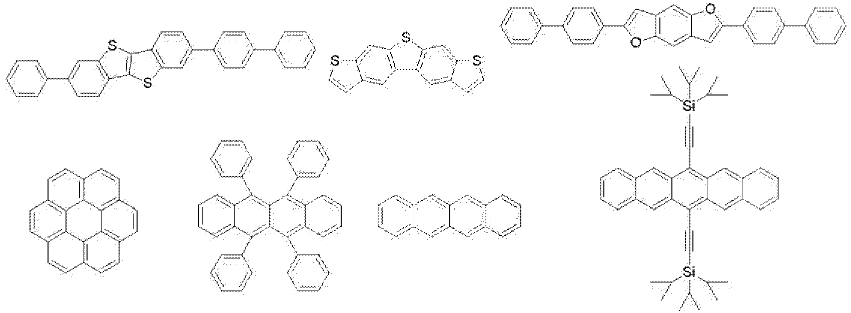

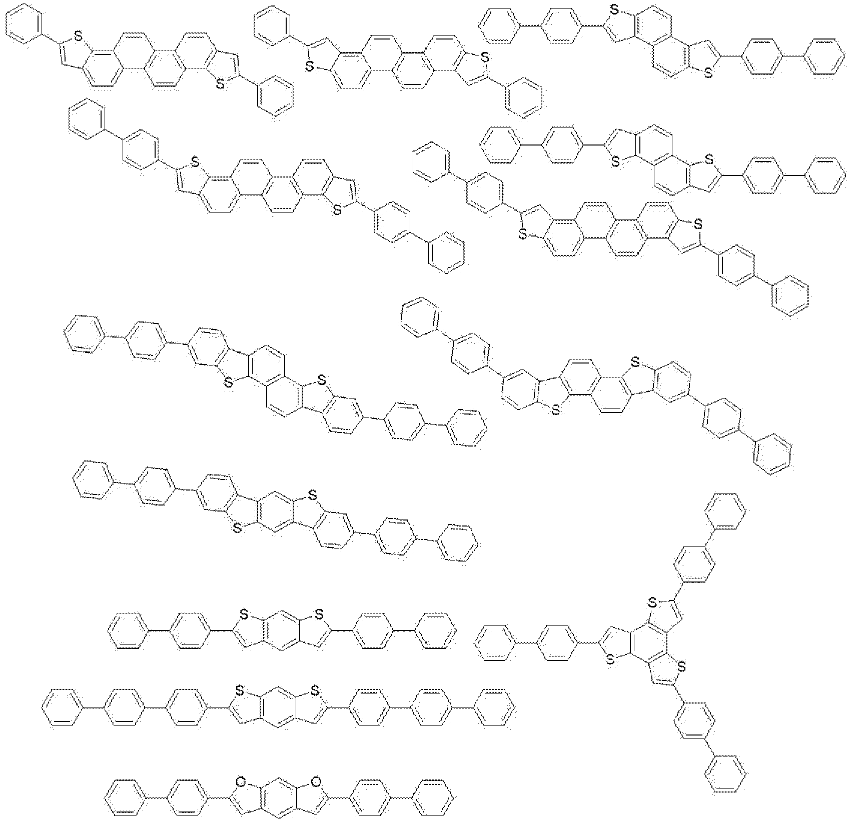

The difference in the ionization potential between the specific compound and the p-type organic semiconductor is preferably 0.1 eV or more.

In a case where the photoelectric conversion film contains the p-type organic semiconductor, a content of the p-type organic semiconductor in the photoelectric conversion film (=film thickness of p-type organic semiconductor in terms of single layer/film thickness of photoelectric conversion film × 100) is preferably 15% to 75% by volume, more preferably 20% to 60% by volume, and still more preferably 25% to 50% by volume.

The p-type semiconductor material may be used alone, or two or more thereof may be used in combination.

The photoelectric conversion film containing the specific compound is a non-light emitting film, and has a feature different from organic light emitting diodes (OLEDs). The non-light emitting film is intended for a film having a light emission quantum efficiency of 1% or less, and the light emission quantum efficiency is preferably 0.5% or less, and more preferably 0.1% or less.

### <Film Formation Method>

The photoelectric conversion film can be formed mostly by a dry film formation method. Examples of the dry film formation method include a physical vapor deposition method such as a vapor deposition method (in particular, a vacuum vapor deposition method), a sputtering method, and an ion plating method, a molecular beam epitaxy (MBE) method, and a chemical vapor deposition (CVD) method such as plasma polymerization. Among these, the vacuum vapor deposition method is preferable. In a case where the photoelectric conversion film is formed by the vacuum vapor deposition method, manufacturing conditions such as a degree of vacuum and a vapor deposition temperature can be set according to the normal method.

The thickness of the photoelectric conversion film is preferably 10 to 1000 nm, more preferably 50 to 800 nm, still more preferably 50 to 500 nm, and particularly preferably 50 to 300 nm.

### [Electrode]

Electrodes (the upper electrode (the transparent conductive film) 15 and the lower electrode (the conductive film) 11) are formed of conductive materials. Examples of the conductive material include metals, alloys, metal oxides, electrically conductive compounds, and mixtures thereof.

Since light is incident through the upper electrode 15, the upper electrode 15 is preferably transparent to light to be detected. Examples of the material forming the upper electrode 15 include conductive metal oxides such as tin oxide (antimony tin oxide (ATO), fluorine doped tin oxide (FTO)) doped with antimony, fluorine, or the like, tin oxide, zinc oxide, indium oxide, indium tin oxide (ITO), and indium zinc oxide (IZO); metal thin films such as gold, silver, chromium, and nickel; mixtures or laminates of these metals and the conductive metal oxides; and organic conductive materials such as polyaniline, polythiophene, and polypyrrole. Among these, conductive metal oxides are preferable from the viewpoints of high conductivity, transparency, and the like.

In general, in a case where the conductive film is made to be thinner than a certain range, a resistance value is rapidly increased. However, in the solid-state imaging element into which the photoelectric conversion element according to the present embodiment is incorporated, the sheet resistance is preferably 100 to 10000 S2/o, and a degree of freedom of a range of the film thickness that can be thinned is large. In addition, as the thickness of the upper electrode (the transparent conductive film) 15 is thinner, the amount of light that the upper electrode absorbs is smaller, and the light transmittance usually increases. The increase in the light transmittance causes an increase in light absorbance in the photoelectric conversion film and an increase in the photoelectric conversion ability, which is preferable. Considering the suppression of leakage current, an increase in the resistance value of the thin film, and an increase in transmittance accompanied by the thinning, the film thickness of the upper electrode 15 is preferably 5 to 100 nm, and more preferably 5 to 20 nm.

There is a case where the lower electrode 11 has transparency or an opposite case where the lower electrode 11 does not have transparency and reflects light, depending on the application. Examples of a material constituting the lower electrode 11 include conductive metal oxides such as tin oxide (ATO, FTO) doped with antimony, fluorine, or the like, tin oxide, zinc oxide, indium oxide, indium tin oxide (ITO), and indium zinc oxide (IZO); metals such as gold, silver, chromium, nickel, titanium, tungsten, and aluminum, conductive compounds (for example, titanium nitride (TiN)) such as oxides or nitrides of these metals; mixtures or laminates of these metals and conductive metal oxides; and organic conductive materials such as polyaniline, polythiophene, and polypyrrole.

The method of forming electrodes is not particularly limited, and can be appropriately selected in accordance with the electrode material. Specific examples thereof include a wet method such as a printing method and a coating method; a physical method such as a vacuum vapor deposition method, a sputtering method, and an ion plating method; and a chemical method such as a CVD method and a plasma CVD method.

In a case where the material of the electrode is ITO, examples thereof include an electron beam method, a sputtering method, a resistance heating vapor deposition method, a chemical reaction method (such as a sol-gel method), and a coating method with a dispersion of indium tin oxide.

### [Charge Blocking Film: Electron Blocking Film and Hole Blocking Film]

It is also preferable that the photoelectric conversion element according to the embodiment of the present invention has one or more interlayers between the conductive film and the transparent conductive film, in addition to the photoelectric conversion film. An example of the interlayer includes a charge blocking film. In a case where the photoelectric conversion element has this film, the characteristics (such as photoelectric conversion efficiency and responsiveness) of the photoelectric conversion element to be obtained is more excellent. Examples of the charge blocking film include an electron blocking film and a hole blocking film. Hereinafter, each of the films will be described in detail.

### <Electron Blocking Film>

The electron blocking film is a donor organic semiconductor material (a compound), and the p-type organic semiconductor described above can be used.

A polymer material can also be used as the electron blocking film.

Examples of the polymer material include a polymer such as phenylenevinylene, fluorene, carbazole, indole, pyrene, pyrrole, picoline, thiophene, acetylene, and diacetylene, and a derivative thereof.

### The electron blocking film may be formed of a plurality of films.

The electron blocking film may be formed of an inorganic material. In general, since an inorganic material has a dielectric constant larger than that of an organic material, in a case where the inorganic material is used in the electron blocking film, a large voltage is applied to the photoelectric conversion film. Therefore, the photoelectric conversion efficiency increases. Examples of the inorganic material that can be used for the electron blocking film include calcium oxide, chromium oxide, copper chromium oxide, manganese oxide, cobalt oxide, nickel oxide, copper oxide, copper gallium oxide, copper strontium oxide, niobium oxide, molybdenum oxide, copper indium oxide, silver indium oxide, and iridium oxide.

### <Hole Blocking Film>

A hole blocking film is an acceptor-property organic semiconductor material (a compound), and the n-type semiconductor described above can be used.

The hole blocking film may be formed of a plurality of films.

The method of producing the charge blocking film is not particularly limited, but a dry film formation method and a wet film formation method are exemplified. Examples of the dry film formation method include a vapor deposition method and a sputtering method. The vapor deposition method may be any of physical vapor deposition (PVD) method and chemical vapor deposition (CVD) method, and physical vapor deposition method such as vacuum vapor deposition method is preferable. Examples of the wet film formation method include an ink jet method, a spray method, a nozzle printing method, a spin coating method, a dip coating method, a casting method, a die coating method, a roll coating method, a bar coating method, and a gravure coating method, and an inkjet method is preferable from the viewpoint of high accuracy patterning.

Each thickness of the charge blocking films (the electron blocking film and the hole blocking film) is preferably 3 to 200 nm, more preferably 5 to 100 nm, and still more preferably 5 to 30 nm.

### [Substrate]

The photoelectric conversion element may further include a substrate. The type of substrate to be used is not particularly limited, but a semiconductor substrate, a glass substrate, and a plastic substrate are exemplified.

The position of the substrate is not particularly limited, but in general, the conductive film, the photoelectric conversion film, and the transparent conductive film are laminated on the substrate in this order.

### [Sealing Layer]

The photoelectric conversion element may further include a sealing layer. The performance of the photoelectric conversion material may deteriorate noticeably due to the presence of deterioration factors such as water molecules. The deterioration can be prevented by coating and sealing the entirety of the photoelectric conversion film with the sealing layer such as diamond-like carbon (DLC) or ceramics such as metal oxide, or metal nitride, and metal nitride oxide which are dense and into which water molecules do not permeate.

The material of the sealing layer may be selected and the sealing layer may be produced according to the description in paragraphs [0210] to [0215] of JP2011-082508A, for example.

### [Imaging Element and Optical Sensor]

An example of the application of the photoelectric conversion element includes an imaging element. The imaging element is an element that converts optical information of an image into an electric signal. In general, a plurality of the photoelectric conversion elements are arranged in a matrix on the same plane, and an optical signal is converted into an electric signal in each photoelectric conversion element (pixel) to sequentially output the electric signal to the outside of the imaging element for each pixel. Therefore, each pixel is formed of one or more photoelectric conversion elements and one or more transistors.

The imaging element is mounted on an imaging element such as a digital camera and a digital video camera, an electronic endoscope, and imaging modules such as a cellular phone.

The photoelectric conversion element according to the embodiment of the present invention is also preferably used for an optical sensor including the photoelectric conversion element of the present invention. The photoelectric conversion element may be used alone as the optical sensor, and the photoelectric conversion element may be used as a line sensor in which the photoelectric conversion elements are linearly arranged or as a two-dimensional sensor in which the photoelectric conversion elements are arranged in a plane shape.

### [Compound]

The present invention further includes the invention of compounds. Examples of the compound according to the embodiment of the present invention include the above-described specific compounds.

### Examples

The present invention will be described in more detail based on Examples below. Materials, used amounts, ratios, treatment contents, treatment procedures, and the like described in the following Examples can be appropriately changed within the range that does not depart from the gist of the present invention. Therefore, the range of the present invention should not be limitatively interpreted by the following Examples.

### [Compound (Evaluation Compound)]

### <Synthesis of Compound (D-1)>

A compound (D-1) serving as a specific compound was synthesized according to the following scheme.

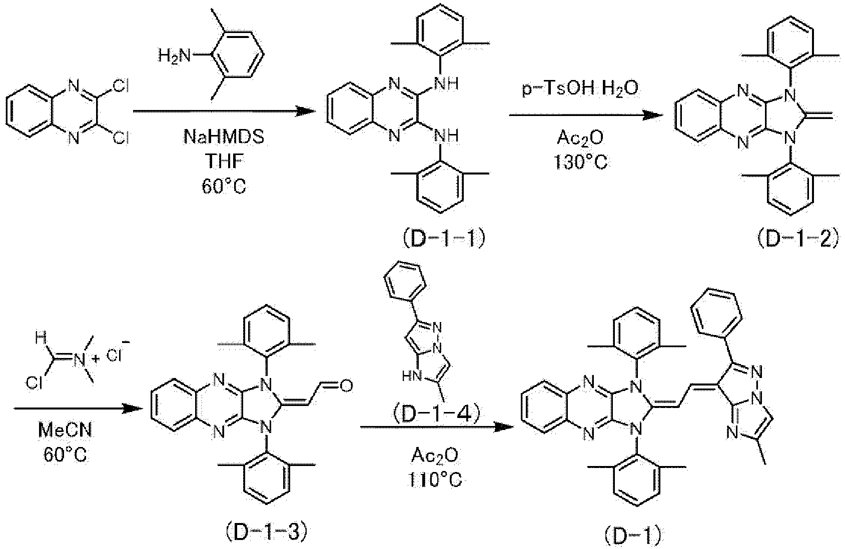

30% hydrochloric acid (288 mL, 2.73 mol) was added dropwise to a mixture of 2,6-xylidine (300 g, 2.48 mol) and isopropanol (300 mL), and the obtained reaction solution was stirred under heating and reflux for 1 hour. The above-described mixed solution was cooled to 2°C, the resulting crystals were filtered off, and the obtained crystals were washed in the order of isopropanol and hexane to obtain 2,6-xylidine hydrochloride (350 g, yield 90%). Subsequently, a 50% aqueous sodium hydroxide solution (199 g, 2.48 mol) was added dropwise to a mixture of 2,6-xylidine hydrochloride (300 g, 1.90 mol) and water (600 mL), and the obtained reaction solution was stirred at room temperature (25°C) for 1 hour. The obtained mixed solution was transferred to a liquid separation funnel, hexane (150 mL) was added thereto, the resulting mixture was washed, and an aqueous phase was then removed. The obtained organic phase was concentrated under reduced pressure to obtain 2,6-xylidine (200 g, 87%).

2,6-xylidine (11 mL, 88.2 mmol) obtained above was added dropwise to a mixture of 2,3-dichloroquinoxalin (8.0 g, 40.2 mmol), hexamethyldisilazane sodium in a 35% tetrahydrofuran solution (1.9 mol/L) (90.4 mL, 181 mol), and tetrahydrofuran (80 mL) to obtain a mixed solution. The mixed solution was stirred at 60°C for 1 hour and left to cool to room temperature (25°C), and water (40 mL) was added dropwise to the mixed solution. Furthermore, 80 mL of saline (20% by mass) was added to the mixed solution, and an organic phase of the mixed solution was then extracted. The obtained organic phase was dried over magnesium sulfate and filtered, and the obtained filtrate was concentrated under reduced pressure. The obtained crude product (residue after concentration under reduced pressure) was purified by recrystallization with toluene/2-propanol to obtain an intermediate (D-1-1) (9.95 g, yield 67%).

p-Toluenesulfonic acid monohydrate (9.3 g, 48.9 mmol), acetic anhydride (16 mL), and the intermediate (D-1-1) (6.0 g, 16.3 mmol) were mixed, and the obtained reaction solution was stirred at 130°C for 1 hour. The reaction solution left to cool to room temperature (25°C) was added dropwise to a mixture of a 50 w/v% aqueous sodium hydroxide solution (48 mL) and ice (143 g) and stirred for 30 minutes to obtain a reaction mixture. Acetic acid was added to the reaction mixture to adjust the pH of the reaction mixture (pH at 25°C) to 8, and the reaction mixture was further stirred for 20 minutes. The precipitate produced in the above reaction mixture was filtered, and the obtained filtration was washed with water and methanol in this order. The obtained crude product (filtration after washing) was purified by reprecipitation with dichloromethane/methanol to obtain an intermediate (D-1-2) (6.2 g, yield 98%).

The intermediate (D-1-2) (6.0 g, 15.3 mmol) was added to a mixture of (chloromethylene)dimethyliminium chloride (5.87 g, 45.9 mmol) and acetonitrile (60 mL) to obtain a reaction solution, and the reaction solution was stirred at 50°C for 2 hours. The reaction solution left to cool to room temperature (25°C) was added dropwise to a mixture of a 1 mol/L aqueous sodium hydroxide solution (90 mL) and ice (90 g), and the obtained mixed solution was stirred for 1 hour. The resulting precipitate produced in the above-described mixed solution was filtered, and the obtained filtration was washed with water and methanol in this order. The obtained crude product (filtration after washing) was purified by reprecipitation with dichloromethane/acetonitrile to obtain an intermediate (D-1-3) (4.4 g, yield 68%).

The intermediate (D-1-4) was synthesized with reference to a known method (for example, the method described in paragraphs [0260] to [0265] of JP2009-235382A).

The intermediate (D-1-3) (0.50 g, 1.19 mmol), the intermediate (D-1-4) (0.31 g, 1.55 mmol), and acetic anhydride (7.5 mL) were mixed, and the obtained reaction solution was stirred at 110°C for 24 hours. The above-described reaction solution was left to cool to room temperature (25°C), the precipitate produced in the reaction solution was filtered, and the obtained filtration was washed with methanol. A crude product (filtration after washing) thus obtained was purified with silica gel chromatography (eluent (volume ratio); dichloromethane/ethyl acetate = 100:0 to dichloromethane/ethyl acetate = 85:15) to obtain a compound (D-1) (0.49 g, yield 68%).

Other specific compounds were also synthesized with reference to the above-described synthesis method.

The specific compound and comparative compound used in a test are shown below.

Hereinbelow, compounds (D-1) to (D-16) are specific compounds.

Hereinafter, the specific compound and the Comparative compound are collectively referred to as an evaluationcompound.

Evaluation compounds were used for producing photoelectric conversion elements described later.

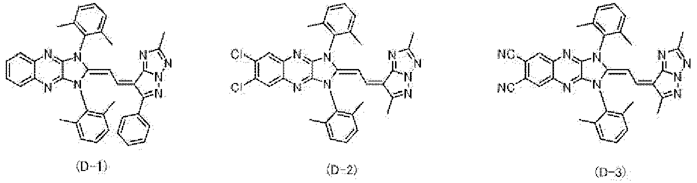

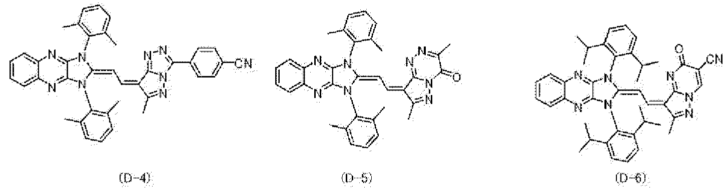

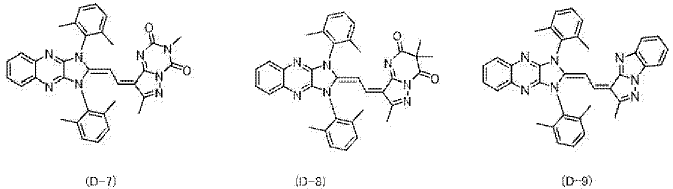

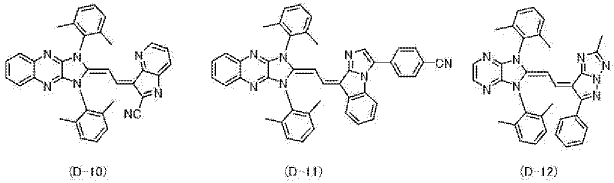

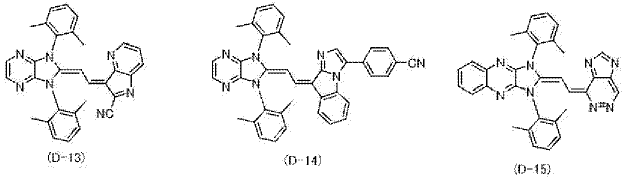

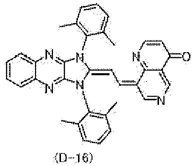

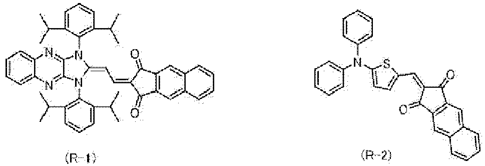

### [n-Type Organic Semiconductor]

Fullerene C₆₀ (C60) was used for the production of photoelectric conversion elements described later, as the n-type organic semiconductor used for evaluations.

### [p-Type Organic Semiconductor]

The p-type organic semiconductor described below was used for producing the photoelectric conversion elements described later, as the p-type organic semiconductor used for evaluations.

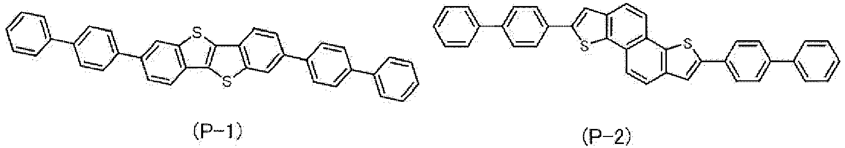

### [Evaluation]

### <Production of Photoelectric Conversion Element>

A photoelectric conversion element having the form illustrated in Fig. 1 was produced using evaluation compounds (specific compounds or comparative compounds). Here, the photoelectric conversion element consists of a lower electrode 11, an electron blocking film 16A, a photoelectric conversion film 12, and an upper electrode 15.

Specifically, an amorphous ITO was formed into a film on a glass substrate by a sputtering method to form the lower electrode 11 (thickness: 30 nm). Furthermore, a compound (EB-1) described below was formed into a film on the lower electrode 11 by a vacuum thermal vapor deposition method to form the electron blocking film 16A (thickness: 30 nm).

Furthermore, in a state where the temperature of the substrate was controlled to 25°C, the evaluation compound, the n-type organic semiconductor (fullerene (C₆₀)), and as desired, the p-type organic semiconductor was co-vapor deposited on the electron blocking film 16A by a vacuum deposition method, each to be 80 nm in terms of a single layer, thereby forming a film. As a result, a photoelectric conversion film 12 having a bulk hetero structure of 160 nm (240 nm in a case where the p-type organic semiconductor was also used) was formed. In this case, a film formation rate of the photoelectric conversion film 12 was set to 1.0 Å/sec.

Furthermore, amorphous ITO was formed into a film on the photoelectric conversion film 12 by a sputtering method to form the upper electrode 15 (the transparent conductive film) (the thickness: 10 nm). After the SiO film was formed as the sealing layer on the upper electrode 15 by a vacuum vapor deposition method, an aluminum oxide (Al₂O₃) layer was formed thereon by an atomic layer chemical vapor deposition (ALCVD) method to produce a photoelectric conversion element.

The obtained photoelectric conversion elements of Examples or Comparative Examples are collectively referred to as an element (A).

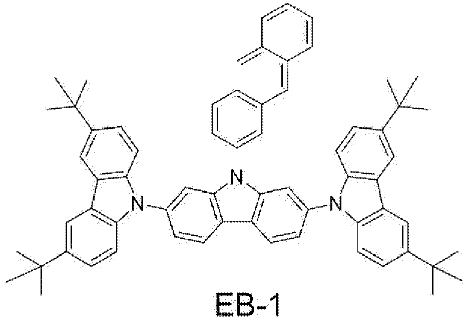

### <Evaluation of Photoelectric Conversion Efficiency (External Quantum Efficiency)>

The drive of each photoelectric conversion element (element (A)) thus obtained was confirmed. A voltage was applied to each photoelectric conversion element to have an electric field strength of 2.0 × 10⁵ V/cm. Thereafter, light is emitted from the upper electrode (transparent conductive film) side to perform an incident photon-to-current conversion efficiency (IPCE) measurement, and the photoelectric conversion efficiency (external quantum efficiency) at each of 500 nm and 600 nm was extracted. The photoelectric conversion efficiency was measured using a constant energy quantum efficiency measuring device manufactured by Optel Co., Ltd.. The amount of light emitted was 50 µW/cm². In a case where the photoelectric conversion efficiency of the photoelectric conversion element of Example 1 was standardized as 1, the photoelectric conversion efficiency of each photoelectric conversion element was obtained and evaluated in the following categories based on the obtained photoelectric conversion efficiency.
AA: 1.1 or more
A: 0.9 or more and less than 1.1
B: 0.8 or more and less than 0.9
C: 0.7 or more and less than 0.8
D: 0.6 or more and less than 0.7
E: less than 0.6

In a case of both wavelengths of 500 nm and 600 nm, AA, A, B, and C are preferable, and AA is most preferable.

In addition, it has been confirmed that the photoelectric conversion elements (element (A)) of Examples or Comparative Examples all exhibited photoelectric conversion efficiencies of 40% or more at measurement wavelengths of 500 nm and 600 nm, and had external quantum efficiencies equal to or higher than a certain level as the photoelectric conversion element.

### <Evaluation of Responsiveness>

The responsiveness of each of the obtained photoelectric conversion elements (element (A)) was evaluated. A voltage was applied to each photoelectric conversion element to have a strength of 2.0 × 10⁵ V/cm. Thereafter, light emitting diodes (LEDs) were turned on momentarily to emit light from the upper electrode (transparent conductive film) side, a photocurrent at a wavelength of 580 nm was measured with an oscilloscope, and a rise time from a signal intensity of 0% (when the light is not emitted) to 97% was calculated. Next, the rise time of each photoelectric conversion element at a wavelength of 580 nm was obtained in a case where the rise time of the photoelectric conversion element of Example 1 was standardized as 1, and the responsiveness of each of the photoelectric conversion elements was evaluated in the following categories based on the obtained rise time.
AA: less than 0.9
A: 0.9 or more and less than 2.0
B: 2.0 or more and less than 3.0
C: 3.0 or more and less than 4.0
D: 4.0 or more and less than 5.0
E: less than 5.0

In practice, AA, A, B, and C are preferable, and AA is most preferable.

### <Evaluation of Manufacturing Suitability (Photoelectric Conversion Efficiency During High-speed Film Formation)>

A photoelectric conversion element (element (B)) of each of Examples or Comparative Examples was produced according to the same procedure as that of the element (A), except that a film formation rate of the photoelectric conversion film 12 was set to 3.0 A/sec. The photoelectric conversion efficiency (external quantum efficiency) of the obtained element (B) was evaluated by the same method as shown in the section of <Evaluation of Photoelectric Conversion Efficiency (External Quantum Efficiency)>.

The photoelectric conversion efficiencies of the element (A) and the element (B) in the same Examples or Comparative Examples were compared to calculate a value of "the photoelectric conversion efficiency of the element (B)/the photoelectric conversion efficiency of the element (A)". The manufacturing suitability of each photoelectric conversion element was evaluated by comparing the obtained values with the following standard.
A: 0.9 or more
B: less than 0.9

The following table shows the types and characteristics of the compounds used to produce the photoelectric conversion films in each photoelectric conversion element (element (A) or element (B)) in the photoelectric conversion element of each of Examples or Comparative Examples.

In addition, the test results are shown in the table below.

In the table, the column of "Formula (4)" indicates whether or not the specific compound used to produce the photoelectric conversion film corresponds to the compound represented by Formula (4). A case where this requirement was satisfied was denoted by "A", and a case where this requirement was not satisfied was denoted by "B".

In the specific compound used to produce the photoelectric conversion film, the column of "Q" indicates whether or not the group corresponding to Q in Formula (1) corresponds to a group represented by any one of Formulae (Q1), (Q2), or (Q3). "Q3" was used in a case where the group corresponds to the group represented by Formula (Q3), and "Q2" was used in a case where the group does not correspond to the group represented by Formula (Q3) and corresponds to the group represented by Formula (Q2), and "Q1" was used in a case where the group only corresponds to the group represented by Formula (Q1).

The "B1" column represents the number of ring member atoms of a ring corresponding to the ring represented by B¹ in Formula (1) in the specific compound used to produce the photoelectric conversion film.

**[Table 1]**

| | Evaluation Compound | | | | n-Type organic semiconductor | p-Type organic semiconductor | Photoelectric conversion efficiency | | Responsiveness | Manufacturing suitability |
|---|---|---|---|---|---|---|---|---|---|---|
| | Type | Characteristics | | | | | | | | |
| | | Formula (4) | Q | B1 | Type | Type | 500 nm | 600 nm | | |
| Example 1 | (D-1) | A | Q3 | 5 | C60 | - | A | A | A | A |
| Example 2 | (D-2) | A | Q3 | 5 | C60 | - | A | A | A | A |
| Example 3 | (D-3) | A | Q3 | 5 | C60 | - | A | A | A | A |
| Example 4 | (D-4) | A | Q3 | 5 | C60 | - | A | A | A | A |
| Example 5 | (D-5) | A | Q3 | 6 | C60 | - | A | A | A | A |
| Example 6 | (D-6) | A | Q3 | 6 | C60 | - | A | A | A | A |
| Example 7 | (D-7) | A | Q3 | 6 | C60 | - | A | A | A | A |
| Example 8 | (D-8) | A | Q3 | 6 | C60 | - | A | A | A | A |
| Example 9 | (D-9) | A | Q3 | 9 | C60 | - | B | A | A | A |
| Example 10 | (D-10) | A | Q2 | 6 | C60 | - | A | A | B | A |
| Example 11 | (D-11) | A | Q1 | 5 | C60 | - | A | B | B | A |
| Example 12 | (D-12) | B | Q3 | 5 | C60 | - | A | B | C | A |
| Example 13 | (D-13) | B | Q2 | 6 | C60 | - | B | B | C | A |
| Example 14 | (D-14) | B | Q1 | 5 | C60 | - | B | C | C | A |
| Example 15 | (D-15) | A | Q1 | 5 | C60 | - | A | B | B | A |
| Example 16 | (D-16) | A | Q1 | 6 | C60 | - | A | B | B | A |
| Example 17 | (D-1) | A | Q3 | 5 | C60 | (P-1) | AA | AA | AA | A |
| Example 18 | (D-1) | A | Q3 | 5 | C60 | (P-2) | AA | AA | AA | A |
| Comparative Example 1 | (R-1) | - | - | - | C60 | - | A | E | B | A |
| Comparative Example 2 | (R-2) | - | - | - | C60 | - | A | B | B | B |
| Comparative Example 3 | (R-1) | - | - | - | C60 | (P-1) | A | E | A | A |

From the results shown in the table, it has been confirmed that the photoelectric conversion element according to the embodiment of the present invention was excellent in the photoelectric conversion efficiency, manufacturing suitability, and responsiveness with respect to a wide range of wavelengths.

In particular, it has been confirmed that the effect of the present invention is more excellent in the case where the specific compound was the compound represented by Formula (4) (see the comparison of results between Example 1 and 10, and other comparisons).

It has been confirmed that in the case where the group represented by Q is the group represented by Formula (Q2) or the group represented by Formula (Q3), the effect of the present invention is more excellent, and in the case where the group represented by Q is the group represented by Formula (Q3), the effect of the present invention is further excellent (see the comparison of results between Examples 1, 8 and 9, and other comparisons).

It has been confirmed that in the case where the number of ring member atoms of the ring represented by B¹ is 5 to 6, the effects of the present invention are further excellent (see the comparison of results between Examples 1 to 7 and other comparisons).

It has been confirmed that in a case where the photoelectric conversion film further contains the p-type organic semiconductor, the effect of the present invention was more excellent (the comparison of results between Examples 1, 13, and 14, and the like).

### Explanation of References

10a, 10b: Photoelectric conversion element
11: Conductive film (lower electrode)
12: Photoelectric conversion film
15: Transparent conductive film (upper electrode)
16A: Electron blocking film
16B: Hole blocking film
20a: Imaging element
22: Blue photoelectric conversion element
24: Red photoelectric conversion element

## Claims

1. A photoelectric conversion element comprising, in the following order:
a conductive film;
a photoelectric conversion film; and
a transparent conductive film,
wherein the photoelectric conversion film contains a compound represented by Formula (1), 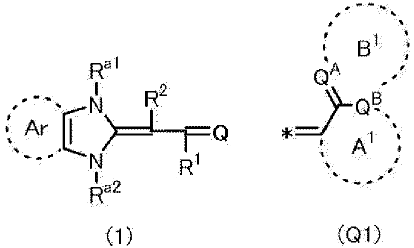
in Formula (1), Ar represents an aromatic ring which may have a substituent,
R¹ and R² each independently represent a hydrogen atom or a substituent,
R^{a1} and R^{a2} each independently represent an aryl group which may have a substituent, -C(R^{L1})(R^{L2})(R^{L3}), or a heteroaryl group which may have a substituent,
R^{L1} to R^{L3} each independently represent an alkyl group which may have a substituent, an aryl group which may have a substituent, a heteroaryl group which may have a substituent, or a hydrogen atom, and at least two of R^{L1}, R^{L2}, or R^{L3} each independently represent an alkyl group which may have a substituent, an aryl group which may have a substituent, or a heteroaryl group which may have a substituent, an alkyl group which may have a substituent, an aryl group which may have a substituent, and a heteroaryl group which may have a substituent, which are represented by R^{L1} to R^{L3}, may be bonded to each other to form a ring, and
Q represents a group represented by Formula (Q1),
in Formula (Q1), * represents a bonding position,
Q^{A} represents a nitrogen atom or -CQ^{X}=, Q^{X} represents a hydrogen atom or a substituent,
Q^{B} represents a nitrogen atom, a group represented by Formula (C), or -CQ^{Y}<, Q^{Y} represents a hydrogen atom or a substituent,
A¹ represents a ring which at least contains two carbon atoms and Q^{B}, and may have a substituent, and
B¹ represents a ring which at least contains one carbon atom, Q^{A}, and Q^{B}, and may have a substituent, 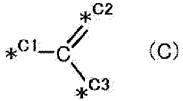
in Formula (C), *^{C1} to *^{C3} each represent a bonding position.

2. The photoelectric conversion element according to claim 1,
wherein the compound represented by Formula (1) is a compound represented by Formula (2), 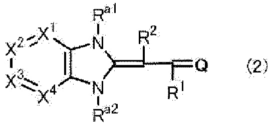
in Formula (2), R¹ and R² each independently represent a hydrogen atom or a substituent,
R^{a1} and R^{a2} each independently represent an aryl group which may have a substituent, -C(R^{L1})(R^{L2})(R^{L3}), or a heteroaryl group which may have a substituent,
R^{L1} to R^{L3} each independently represent an alkyl group which may have a substituent, an aryl group which may have a substituent, a heteroaryl group which may have a substituent, or a hydrogen atom, and at least two of R^{L1}, R^{L2}, or R^{L3} each independently represent an alkyl group which may have a substituent, an aryl group which may have a substituent, or a heteroaryl group which may have a substituent, an alkyl group which may have a substituent, an aryl group which may have a substituent, and a heteroaryl group which may have a substituent, which are represented by R^{L1} to R^{L3}, may be bonded to each other to form a ring,
Q represents the group represented by Formula (Q1),
X¹ to X⁴ each independently represent a nitrogen atom or -CR^{c1}=,
R^{c1} represents a hydrogen atom or a substituent, and
in a case where a plurality of R^{c1}'s exist, the plurality of R^{c1}'s may be bonded to each other to form a ring.

3. The photoelectric conversion element according to claim 1 or 2,
wherein the compound represented by Formula (1) is a compound represented by Formula (3), 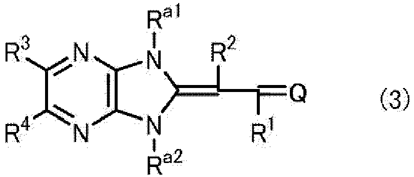
in Formula (3), R¹ to R⁴ each independently represent a hydrogen atom or a substituent,
R³ and R⁴ may be bonded to each other to form a ring,
R^{a1} and R^{a2} each independently represent an aryl group which may have a substituent, -C(R^{L1})(R^{L2})(R^{L3}), or a heteroaryl group which may have a substituent,
R^{L1} to R^{L3} each independently represent an alkyl group which may have a substituent, an aryl group which may have a substituent, a heteroaryl group which may have a substituent, or a hydrogen atom, and at least two of R^{L1}, R^{L2}, or R^{L3} each independently represent an alkyl group which may have a substituent, an aryl group which may have a substituent, or a heteroaryl group which may have a substituent, an alkyl group which may have a substituent, an aryl group which may have a substituent, and a heteroaryl group which may have a substituent, which are represented by R^{L1} to R^{L3}, may be bonded to each other to form a ring, and
Q represents the group represented by Formula (Q1).

4. The photoelectric conversion element according to any one of claims 1 to 3,
wherein the compound represented by Formula (1) is a compound represented by Formula (4), 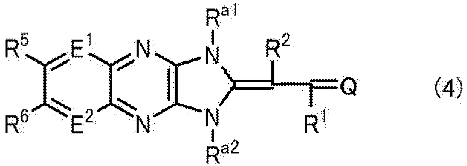
in Formula (4), R¹, R², R⁵, and R⁶ each independently represent a hydrogen atom or a substituent,
R⁵ and R⁶ may be bonded to each other to form a ring,
R^{a1} and R^{a2} each independently represent an aryl group which may have a substituent, -C(R^{L1})(R^{L2})(R^{L3}), or a heteroaryl group which may have a substituent,
R^{L1} to R^{L3} each independently represent an alkyl group which may have a substituent, an aryl group which may have a substituent, a heteroaryl group which may have a substituent, or a hydrogen atom, and at least two of R^{L1}, R^{L2}, or R^{L3} each independently represent an alkyl group which may have a substituent, an aryl group which may have a substituent, or a heteroaryl group which may have a substituent, an alkyl group which may have a substituent, an aryl group which may have a substituent, and a heteroaryl group which may have a substituent, which are represented by R^{L1} to R^{L3}, may be bonded to each other to form a ring,
Q represents the group represented by Formula (Q1),
E¹ represents a nitrogen atom or -CR^{E1}=, R^{E1} represents a hydrogen atom or a substituent,
E² represents a nitrogen atom or -CR^{E2}=, and R^{E2} represents a hydrogen atom or a substituent.

5. The photoelectric conversion element according to any one of claims 1 to 4,
wherein Q represents a group represented by Formula (Q2), 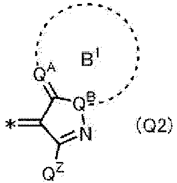
in Formula (Q2), * represents a bonding position,
Q^{A} represents a nitrogen atom or -CQ^{X}=, Q^{X} represents a hydrogen atom or a substituent,
Q^{B} represents a nitrogen atom, the group represented by Formula (C), or -CQ^{Y}<, Q^{Y} represents a hydrogen atom or a substituent,
Q^{Z} represents a hydrogen atom or a substituent, and
B¹ represents a ring which at least contains one carbon atom, Q^{A}, and Q^{B}, and may have a substituent.

6. The photoelectric conversion element according to any one of claims 1 to 5,
wherein Q represents a group represented by Formula (Q3), 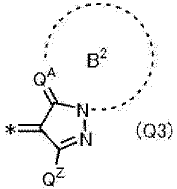
in Formula (Q3), * represents a bonding position,
Q^{A} represents a nitrogen atom or -CQ^{X}=, Q^{X} represents a hydrogen atom or a substituent,
Q^{Z} represents a hydrogen atom or a substituent, and
B² represents a ring which at least contains one carbon atom, one nitrogen atom, and Q^{A}, and may have a substituent.

7. The photoelectric conversion element according to any one of claims 1 to 6,
wherein the photoelectric conversion film further includes a n-type organic semiconductor, and
the photoelectric conversion film has a bulk hetero structure formed in a state where the compound represented by Formula (1) and the n-type organic semiconductor are mixed to each other.

8. The photoelectric conversion element according to claim 7,
wherein the n-type organic semiconductor includes fullerenes selected from the group consisting of a fullerene and a derivative thereof.

9. The photoelectric conversion element according to claim 7 or 8,
wherein the photoelectric conversion film further contains a p-type organic semiconductor.

10. The photoelectric conversion element according to any one of claims 1 to 9, further comprising one or more interlayers between the conductive film and the transparent conductive film, in addition to the photoelectric conversion film.

11. An imaging element comprising the photoelectric conversion element according to any one of claims 1 to 10.

12. An optical sensor comprising the photoelectric conversion element according to any one of claims 1 to 10.

13. A compound represented by Formula (1), 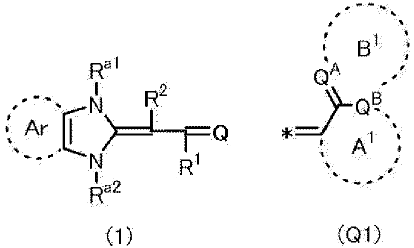
in Formula (1), Ar represents an aromatic ring which may have a substituent,
R¹ and R² each independently represent a hydrogen atom or a substituent,
R^{a1} and R^{a2} each independently represent an aryl group which may have a substituent, -C(R^{L1})(R^{L2})(R^{L3}), or a heteroaryl group which may have a substituent,
R^{L1} to R^{L3} each independently represent an alkyl group which may have a substituent, an aryl group which may have a substituent, a heteroaryl group which may have a substituent, or a hydrogen atom, and at least two of R^{L1}, R^{L2}, or R^{L3} each independently represent an alkyl group which may have a substituent, an aryl group which may have a substituent, or a heteroaryl group which may have a substituent, an alkyl group which may have a substituent, an aryl group which may have a substituent, and a heteroaryl group which may have a substituent, which are represented by R^{L1} to R^{L3}, may be bonded to each other to form a ring, and
Q represents a group represented by Formula (Q1),
in Formula (Q1), * represents a bonding position,
Q^{A} represents a nitrogen atom or -CQ^{X}=, Q^{X} represents a hydrogen atom or a substituent,
Q^{B} represents a nitrogen atom, a group represented by Formula (C), or -CQ^{Y}<, Q^{Y} represents a hydrogen atom or a substituent,
A¹ represents a ring which at least contains two carbon atoms and Q^{B}, and may have a substituent, and
B¹ represents a ring which at least contains one carbon atom, Q^{A}, and Q^{B}, and may have a substituent, 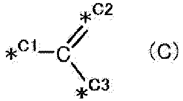
in Formula (C), *^{C1} to *^{C3} each represent a bonding position.

14. The compound according to claim 13,
wherein the compound represented by Formula (1) is a compound represented by Formula (2), 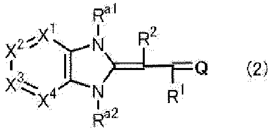
in Formula (2), R¹ and R² each independently represent a hydrogen atom or a substituent,
R^{a1} and R^{a2} each independently represent an aryl group which may have a substituent, -C(R^{L1})(R^{L2})(R^{L3}), or a heteroaryl group which may have a substituent,
R^{L1} to R^{L3} each independently represent an alkyl group which may have a substituent, an aryl group which may have a substituent, a heteroaryl group which may have a substituent, or a hydrogen atom, and at least two of R^{L1}, R^{L2}, or R^{L3} each independently represent an alkyl group which may have a substituent, an aryl group which may have a substituent, or a heteroaryl group which may have a substituent, an alkyl group which may have a substituent, an aryl group which may have a substituent, and a heteroaryl group which may have a substituent, which are represented by R^{L1} to R^{L3}, may be bonded to each other to form a ring,
Q represents the group represented by Formula (Q1),
X¹ to X⁴ each independently represent a nitrogen atom or -CR^{c1}=,
R^{c1} represents a hydrogen atom or a substituent, and
in a case where a plurality of R^{c1}'s exist, the plurality of R^{c1}'s may be bonded to each other to form a ring.

15. The compound according to claim 13 or 14,
wherein the compound represented by Formula (1) is a compound represented by Formula (3), 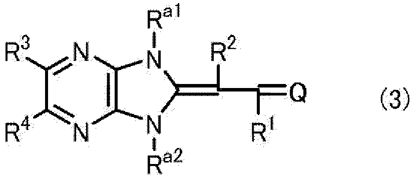
in Formula (3), R¹ to R⁴ each independently represent a hydrogen atom or a substituent,
R³ and R⁴ may be bonded to each other to form a ring,
R^{a1} and R^{a2} each independently represent an aryl group which may have a substituent, -C(R^{L1})(R^{L2})(R^{L3}), or a heteroaryl group which may have a substituent,
R^{L1} to R^{L3} each independently represent an alkyl group which may have a substituent, an aryl group which may have a substituent, a heteroaryl group which may have a substituent, or a hydrogen atom, and at least two of R^{L1}, R^{L2}, or R^{L3} each independently represent an alkyl group which may have a substituent, an aryl group which may have a substituent, or a heteroaryl group which may have a substituent, an alkyl group which may have a substituent, an aryl group which may have a substituent, and a heteroaryl group which may have a substituent, which are represented by R^{L1} to R^{L3}, may be bonded to each other to form a ring, and
Q represents the group represented by Formula (Q1).

16. The compound according to any one of claims 13 to 15,
wherein the compound represented by Formula (1) is a compound represented by Formula (4), 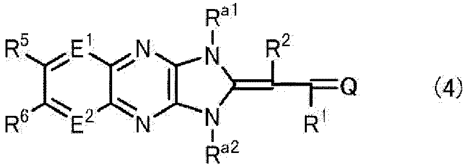
in Formula (4), R¹, R², R⁵, and R⁶ each independently represent a hydrogen atom or a substituent,
R⁵ and R⁶ may be bonded to each other to form a ring,
R^{a1} and R^{a2} each independently represent an aryl group which may have a substituent, -C(R^{L1})(R^{L2})(R^{L3}), or a heteroaryl group which may have a substituent,
R^{L1} to R^{L3} each independently represent an alkyl group which may have a substituent, an aryl group which may have a substituent, a heteroaryl group which may have a substituent, or a hydrogen atom, and at least two of R^{L1}, R^{L2}, or R^{L3} each independently represent an alkyl group which may have a substituent, an aryl group which may have a substituent, or a heteroaryl group which may have a substituent, an alkyl group which may have a substituent, an aryl group which may have a substituent, and a heteroaryl group which may have a substituent, which are represented by R^{L1} to R^{L3}, may be bonded to each other to form a ring,
Q represents the group represented by Formula (Q1),
E¹ represents a nitrogen atom or -CR^{E1}=, R^{E1} represents a hydrogen atom or a substituent,
E² represents a nitrogen atom or -CR^{E2}=, and R^{E2} represents a hydrogen atom or a substituent.

17. The compound according to any one of claims 13 to 16,
wherein Q represents a group represented by Formula (Q2), 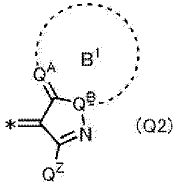
in Formula (Q2), * represents a bonding position,
Q^{A} represents a nitrogen atom or -CQ^{X}=, Q^{X} represents a hydrogen atom or a substituent,
Q^{B} represents a nitrogen atom, the group represented by Formula (C), or -CQ^{Y}<, Q^{Y} represents a hydrogen atom or a substituent,
Q^{Z} represents a hydrogen atom or a substituent, and
B¹ represents a ring which at least contains one carbon atom, Q^{A}, and Q^{B}, and may have a substituent.

18. The compound according to any one of claims 13 to 17,
wherein Q represents a group represented by Formula (Q3), 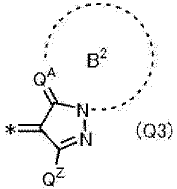
in Formula (Q3), * represents a bonding position,
Q^{A} represents a nitrogen atom or -CQ^{X}=, Q^{X} represents a hydrogen atom or a substituent,
Q^{Z} represents a hydrogen atom or a substituent, and
B² represents a ring which at least contains one carbon atom, one nitrogen atom, and Q^{A}, and may have a substituent.
